# EUROPEAN PATENT APPLICATION

(11) **EP 3 480 320 A1**
(43) Date of publication of application: **08.05.2019**
(21) Application number: 17820585.2
(22) Date of filing: 30.06.2017
(51) Int. Cl.: C12Q 1/68, C12Q 1/70

(54) **DOUBLE-STRANDED NUCLEIC ACID SIGNAL PROBE AND METHOD FOR DETECTING TARGET MOLECULE USING SAME**

(30) Priority: 30.06.2016 KR 20160082612
(71) Applicant: Biois Co., Ltd., Seoul 08390 (KR)
(72) Inventor: KIM, Sung Chun, Seodaemun-gu Seoul 03733 (KR)
(74) Representative: Betten & Resch
(86) International application number: PCT/KR2017/006987
(87) International publication number: WO 2018/004309

(57) **Abstract**

Disclosed is a stable double-stranded nucleic acid signal probe in which single-stranded nucleic acids constituting a double-stranded nucleic acid are complementarily crosslinked (or covalently bonded) with each other, and thus are not affected by temperature, pH, salinity, ionic strength, etc. Also disclosed is a method capable of detecting a target molecule by forming a complex of the stable double-stranded nucleic acid signal probe and the target molecule using the signal probe in the detection of the target molecule, separating only the signal probe from the complex by heating or the like, and detecting the signal probe.

## Description

### Technical Field

The present invention relates to a double-stranded nucleic acid signal probe and a method of detecting a target molecule using the same.

### Background Art

Genetic information, encoded in cellular DNA, is passed from one generation to the next through DNA replication. The genetic information is transferred through DNA to RNA and then to protein. All types of cells are composed of biomolecules, which are associated with transmission to offspring or expression of genetic information, and the expression patterns of biomolecules have large effects on the functions of organisms.

Target molecules (or biomarkers) are biomolecules that are clinically detectable and have been chosen for particular uses. Biomolecules, depending on the onset or progress of diseases, can exhibit an increased or decreased expression within cells or can be expressed by being secreted, and also can easily spread through the bloodstream from specific tissues into terminal tissues. Also, altered expression of biomolecules is detected when cells are destroyed.

Representative examples of biomolecules are nucleic acids and amino acids. Nucleic acids are composed of DNA, carrying genetic information, and RNA, serving as an intermediator for the flow of genetic information, and the expression of genetic information is affected by chromosomal abnormalities, DNA methylation, gene variations, or the like. Among them, common gene variations are single nucleotide polymorphisms (SNPs) and structural variations. SNPs are DNA sequence changes or variations that occur when a single nucleotide (A, T, G or C) in the genome sequence is altered. Structural variations are the genetic variations in the structure of chromosomes with different types of rearrangements, such as deletions, inversions, insertions, and duplications. Gene variations are known to contribute to individual differences, including variability in phenotypes and differences in susceptibility to diseases and in response to drug treatment. In particular, variations involved in the onset and progress of diseases are referred to as disease-associated genetic variants.

The gene is a unit of hereditary information that is a factor determining phenotypic traits. Each cell contains about 50,000 to 100,000 genes, but genes are selectively used in each cell. Most of them are genes that are needed for the survival of the cell itself or daily activities. Endogenous reference genes are commonly used to normalize expression levels of other specific or multiple genes and are based on the level of messenger RNA (hereinafter referred to as "mRNA"). The quantitative analysis of biological samples is important in terms of identifying the functions of specific genes, screening for genes responsible for specific functions, profiling the gene expression patterns under specific conditions and several other molecular biological purposes, and various assays for assessing gene expression have been developed based on endogenous reference genes.

A representative method for detecting gene expression is DNA microarray analysis, which is a high-throughput technology. However, DNA microarrays, which are adapted to detect target sequences only through hybridization, can yield false positive results due to cross reactions, and thus the reliability of hybridization signals needs to be improved. Also, conventional DNA microarrays, which are designed to detect nucleic acids in a sample through hybridization, require stringent post-hybridization washing steps, and a target sequence needs to be denatured to a single strand prior to hybridization. Besides, on-chip PCR, which has been recently developed as a heterogeneous assay system, like conventional microarrays, and which is based on detection through hybridization or probe extension, does not enable real-time detection and is difficult to use to perform accurate quantitation.

Biomolecules include, as well as nucleic acids, amino acids, peptides, proteins, carbohydrates, lipids, polysaccharides, glycoproteins, hormones, receptors, antigens, antibodies, viruses, pathogens, toxins, substrates, metabolites, transition-state analogs, cofactors, inhibitors, drugs, dyes, nutrients, growth factors, cells, and tissues, but are theoretically not limited thereto. Biomolecules encompass almost all chemical or biological effectors and have a variety of sizes. There is a need for effective real-time detection and accurate quantitation of such biomolecules.

Advances in technology for analyzing biomolecules have enhanced the detection sensitivity and multiplexing capability. However, these improved techniques still face problems with respect to lower cost, faster processing time, better sensitivity and better reproducibility.

Meanwhile, a fluorescent barcode probe, which allows molecular counting of target biomolecules, is disclosed in U.S. Pat. No. 8,519,115 (related published article: Nat. Biotechnol. 2008, 26:317-325). The fluorescent barcode probe comprises a signal-generating region, which comprises seven RNA segments that are annealed to a complementary DNA backbone and are arranged in a continuous linear combination; and a target molecule-binding region, which is a single-stranded nucleic acid that is hybridized in a specific manner to a target nucleic acid. The fluorescent barcode probe binds to a target nucleic acid to form a complex and generates a detectable signal specific to a target molecule, and thus enables the complex (i.e., the target molecule) to be individually counted. Since the fluorescent barcode probe is a DNA-RNA double-stranded nucleic acid in which the two strands are joined together via hydrogen bonds between base pairs, its stability is affected by temperature, pH, salinity, ionic strength, and the like. That is, when the double-stranded nucleic acids of the probe are separated due to temperature or the like, the fluorescent barcode probe cannot provide accurate information on a target molecule. Therefore, the method of detecting a target molecule disclosed in US Pat. No. 8,519,115 has an inherent limitation in that temperature, pH, salinity, ionic strength, etc. have to be taken into consideration.

The present invention has been made to overcome this limitation. The present invention provides a double-stranded nucleic acid probe, in which single-stranded nucleic acids are complementarily joined to form a double-stranded nucleic acid through complementary covalent bonds to thus not be affected by temperature, pH, salinity, ionic strength, etc.

### Disclosure

### Technical Problem

Accordingly, the present invention has been made keeping in mind the above problems occurring in the related art, and it is therefore an object of the present invention to provide a stable double-stranded nucleic acid signal probe, in which single-stranded nucleic acids are complementarily joined together to form a double-stranded nucleic acid through cross-linking (or covalent bonding) to thus not be affected by temperature, pH, salinity, ionic strength, or the like.

It is another object of the present invention to provide a method of detecting a target molecule using such a double-stranded nucleic acid signal probe.

Other objects or detailed objects of the present invention are provided below.

### Technical Solution

In an aspect of the present invention, there is provided a double-stranded nucleic acid signal probe.

The double-stranded nucleic acid signal probe, as described above, is characterized in that single-stranded nucleic acids are complementarily joined together to form a double-stranded nucleic acid through cross-linking or covalent bonding.

In detail, the double-stranded nucleic acid signal probe of the present invention comprises (i) a region that binds specifically to a target molecule (in some cases, the "region" may be also represented as a "moiety" for clear distinction of the same or similar terms in other portions); and (ii) a signal-generating region, which is linked to the target-molecule-specific binding region and generates an innate (or specific) signal of the signal probe, wherein the signal-generating region is a double-stranded nucleic acid formed via complementary cross-linking (or covalent bonding), and the double-stranded nucleic acid contains two or more regions, each of which is labelled with the same or a different signal-generating substance (in some cases, the "region" may be also represented as a "segment" for clear distinction of the same or similar terms in other portions).

In the signal probe of the present invention, the region specifically binding to a target molecule may be, as representative examples, a single-stranded nucleic acid, an antibody, or an aptamer. When the target-specific binding region is composed of a single-stranded nucleic acid, its target molecule may be a nucleic acid such as mRNA or the like. When the region is an antibody, its target molecule may be an antigen having an epitope. When the region is an aptamer, its target molecule may be a protein, such as an antigen or the like, which the aptamer specifically recognizes and binds to.

The single-stranded nucleic acid, serving as the region specifically binding to a target molecule, may be, as well as single-stranded DNA or single-stranded RNA, nucleic acid analogs, as long as it has the ability to bind specifically to its target molecule, nucleic acids such as mRNA or the like, and exemplary nucleic acid analogs include Peptide Nucleic Acids (PNA), Locked Nucleic Acids (LNA), Hexitol Nucleic Acids (HNA), Altritol Nucleic Acids (ANA) and Mannitol Nucleic Acids (MNA). Likewise, as long as it has an ability to bind specifically to its target molecule nucleic acid, such as mRNA, the single-stranded nucleic acid is not limited to natural nucleotides and may include modified nucleotides. Such modified nucleotides may be modified at the sugar, phosphate and/or base moiety. The nucleotides modified at the sugar, phosphate and/or base moiety, including their manufacturing methods, are known in detail in the art. The modifications at the sugar moiety, for example, comprise modified sugars in which a hydroxyl group (OH group) is modified with a halogen group, an aliphatic group, an ether group, an amine group, and the like. Nucleotides can also contain analogous forms of ribose or deoxyribose sugars, wherein the ribose or deoxyribose sugars are substituted with, for example, α-anomeric sugars, epimeric sugars such as arabinose, xyloses or lyxoses, pyranose sugars and furanose sugars. Also, nucleotides can be modified at the phosphate moiety in which phosphate is replaced by, for example, P(O)S (thioate), P(S)S (dithioate), P(O)NR'2 (amidate), P(O)R, P(O)OR', CO or CH2 (formacetal), in which each R or R' is independently H or a substituted or unsubstituted alkyl. When phosphate is modified, adjacent nucleotides are linked to each other through an -O-, -N-, -S-or -C- linkage.

The nucleic acid analogs or modified nucleotides, the single-stranded nucleic acid containing such modified nucleotides and the like are known in the art and described in numerous references, for example, in Sproat, et al., Nucl. Acid Res. 19:733-738 (1991), Cotten, et al., Nucl. Acid Res. 19:2629-2635 (1991), and Hobbs, et al., Biochemistry 12:5138-5145 (1973), and reference is to be made thereto for more details. Including these references, all of the references cited throughout this application are to be taken as a part of this application.

The single-stranded nucleic acid serving as the target-molecule-specific binding region is not specifically limited in length as long as it can retain its specific ability to bind to a target molecule. The nucleic acid generally comprises from 5 to 70 nucleotides. However, since longer or shorter probes can cause non-specific binding, the length may preferably range from 15 to 50 bases.

The antibody serving as the target-molecule-specific binding region may be, as well as a monoclonal antibody or a polyclonal antibody, a multispecific antibody (that is, an antibody that recognizes two or more antigens or epitopes, such as a bispecific antibody), or may be an antibody fragment, a chemically modified antibody, a humanized antibody, a recombinant antibody, and the like, as long as it retains the ability to bind specifically to a target molecule. Various forms of antibody fragments and chemically modified antibodies are known in the art, and include, for example, Fab, F(ab')₂, scFv (an antibody in which Fv regions of the heavy chain and the light chain are connected by an appropriate linker), Fv and Fab/c (consisting of one Fab arm and the complete Fc region), as well as antibody fragments, which are produced from intact antibodies, for example by proteolytic cleavage with enzymes such as papain or pepsin.

The aptamer serving as the target-molecule-specific binding region may be a single-stranded DNA aptamer or a single-stranded RNA aptamer. The aptamer, like the antibody, refers to a nucleic acid ligand that is capable of binding specifically to a target molecule, such as a target protein. As long as it retains the ability to bind specifically to a target molecule, the aptamer may be a double-stranded DNA or RNA aptamer. Methods for manufacturing and selecting such an aptamer having the ability to bind specifically to a target molecule are known in the art, and, in particular, SELEX technology may be used. SELEX is an acronym for Systematic Evolution of Ligands by EXponential enrichment, which is described in Science 249 (4968):505-510, 1990, U.S. Pat. Nos. 5,475,096 and 5,270,163, International PCT Publication No. WO 91/19813, and the like. The use of detailed methods or appropriate reagents, materials and others for aptamer selection are found, for example, in Methods Enzymol. 267:275-301, 1996, and Methods Enzymol. 318:193-214, 2000. The aptamer may be a modified form from sugars, phosphates and/or bases thereof.

The target-molecule-specific binding region is representatively a single-stranded nucleic acid, an antibody or an aptamer, but may be an antigen for detecting an antibody, as a target molecule, to an invading antigen in a biological sample. The invading antigen is derived from bacteria, viruses, etc., which have invaded the body, etc., and the detection of antibodies to such invading antigens may be used for detecting the infection of bacteria and viruses.

In addition, Fc fragments of antibodies, peptides, peptidomimetics, gapmers and the like may be used as the target-molecule-specific binding region without particular limitation as long as they have the ability to bind specifically to a target molecule.

The target molecule that can be detected by the signal probe of the present invention is a certain molecule that can be specifically recognized and bound by the target-molecule-specific binding region of the signal probe, such as the aforementioned single-stranded nucleic acid, antibody, aptamer, antigen or the like, which is capable of binding specifically the target molecule. The molecule is preferably a biological molecule from, for example, humans or animals, and is more preferably a molecule from humans, among biological molecules. Further preferred may be a biomarker that is useful for the diagnosis of diseases. Representative examples of the molecule include nucleic acids such as mRNA, antigens derived from bacteria, viruses, etc. or antibodies to such antigens. Moreover, the target molecule may be a surface protein of a bacteria or viruses, which is capable of binding specifically to the target-molecule-specific binding region of the signal probe of the present invention, thereby allowing bacterial or viral detection.

In the signal probe of the present invention, the signal-generating region is a double-stranded nucleic acid, in which two strands are cross-linked to each other and are thus stably kept in the double-stranded form even at a high temperature and not affected by pH, salinity, ionic strength, etc. This overcomes a drawback in which a double-stranded nucleic acid dissociates into single strands by a change such as a temperature change during a process of detecting a target molecule, thus causing the loss in a single-strand nucleic acid, which generates a detectable signal, and thereby reducing the detection accuracy for the target molecule. Also, the stable double-stranded form makes it possible to detect a target molecule when the signal probe of the present invention is used for detecting a target molecule as described below by forming a complex of the signal probe of the present invention with the target molecule, separating the complex, heating the complex, and detecting only the signal probe separated from the complex.

In the present invention, the cross-linking between two strands of the signal-generating region may be induced using a method known in the art. In detail, the cross-linking may be achieved by reacting the hybridized duplex with psoralen, known as an intercalator between double strands of nucleic acid, psoralen derivatives, such as or 8-methoxypsoralen, mitomycin C, pyrrolobenzodiazepine, melphalan, cyclophosphamide, ethidium bromide, proflavine, daunomycin, doxorubicin, thalidomide, and the like, and exposing the same to UV light at an appropriate wavelength. In an embodiment of the present invention, when the interstrand cross-linking is induced using 8-methoxypsoralen as an intercalator following by UV irradiation at 365 nm, the duplex was found to be stably maintained even at 95°C.

In the present invention, the signal-generating substance (i.e., a detectable label) of the signal-generating region is a certain material that generates a detectable signal and is known in the art. Such a material covalently or non-covalently binds to the single strand or double strands of a nucleic acid fragment (or a nucleotide, which is a basic unit that constitutes the nucleic acid fragment), constituting the signal-generating region.

The signal-generating substance (detectable label) may be any one of various known materials including fluorescent substances, chemiluminescent compounds and radioactive isotopes. Examples of the fluorescent materials include cyanine fluorescent dyes (Cy2, Cy3, or Cy5), Alexa Fluor fluorescent dyes (Alexa Fluor 350, 405, 430, 488, 514, 594, 610 or 647, ThermoFisher Scientific, USA), fluorescein isothiocyanate, tetramethyl rhodamine isothiocyanate, substituted rhodamine isothiocyanate, dichlorotriazine isothiocyanate, and the like. Examples of chemiluminescent compounds include luminol, isoluminol, luciferin, lucigenin, disodium 3-(4-methoxyspiro {1,2-dioxetane-3,2'-(5'-chloro)tricyclo[3.3.1.13,7]decan}-4-yl) phenyl phosphate dioxetane (CSPD), 3-(2'-Spiroadamantane)-4-methoxy-4-(3"-phosphoryloxy)phenyl-1,2-dioxetane (AMPPD), and the like. Examples of radioactive isotopes include tritium, iodine isotopes (¹³¹I, ¹²⁵I, ¹²³I, ¹²¹I), Phosphorus-32 (³²P), Sulfur-35 (³⁵S), radioactive metals (e.g., ⁶⁸Ga, ⁶⁷Ga, ⁶⁸Ge, ⁵⁴Mn, ⁹⁹Mo, ⁹⁹Tc, and ¹³³Xe), and the like. Other examples of the signal-generating substances (detectable labels) including fluorescent materials are described in, for example, International PCT Publications WO 92/15673, WO 95/07463, WO 98/14605, WO 98/26277 and WO 99/49019, as well as in U.S. Pat. Nos. 5,292,658, 5,418,155, 5,683,888, 5,741,668, 5,777,079 and 5,876,995.

Preferably, used are cyanine fluorescent dyes or Alexa Fluor fluorescent dyes, each of which has a different color. In general, when a nucleic acid is labeled with cyanine fluorescent dyes or Alexa Fluor fluorescent dyes, the nucleic acid, as is known in the art, is amplified with any particular one among four aminoallyl nucleotides (any one of four nucleotides, e.g., aminoallyl-dCTP or aminoallyl-UTP) or more aminoallyl nucleotides so that one or more aminoallyl nucleotides are incorporated into the nucleic acid strand at a constant interval (e.g., at the dCTP position when aminoallyl-dCTP is used), followed by labeling of an amine group of the particular one or more aminoallyl nucleotides through coupling (covalent bonding) with a fluorescent dye. In an exemplary embodiment of the present invention, a nucleic acid is amplified using aminoallyl-dCTP, and the aminoallyl-dCTP is then coupled to four different fluorescent dyes including Alexa Fluor 488 (blue) and Cy3 (green), thereby labeling the nucleic acid.

In the present invention, the signal-generating region produces a signal unique to the signal probe containing the signal-generating region. Because the signal probe has the target-molecule-specific binding region, this unique signal that the signal-generating region produces is a signal unique to the target molecule. Thus, when the signal-generating region consists of only one region assigned by a single nucleic acid fragment and becomes thus labeled with only one signal-generating substance (detectable label), the unique signal generated from the signal-generating substance is extremely limited depending on the number of available types of signal-generating substances. For this reason, it is preferred that the signal-generating region consist of at least two or more regions. When the signal-generating region consists of two regions, if labeled with fluorescent dyes capable of generating three different signals (e.g., Alexa Fluor 488 (blue), Cy3 (green) and Cy5 (red)), the linear order of signals generated from each region creates 9 (=3²) unique signals, thus increasing the number of detectable, distinguishable target molecules to nine. In an embodiment of the present invention, the signal-generating region consists of six regions, each of which is labeled with one of four different fluorescent dyes to thus give a signal-generating region producing a total of 4,096 (=4⁶) different signals (that is, 4,096 different signal probes containing signal-generating regions, each of which produces a different signal). U.S. Pat. No. 8,519,115 (its related published article: Nat. Biotechnol. 2008, 26:317-325) discloses in an embodiment thereof that a signal-generating region consists of a total of 7 regions, each of which is labeled with one of four different fluorescent dyes, to thus create a population of 16,384 (=4⁶) signal probes. The linear order of signals produced from each region of a signal-generating region may be quantified by collecting images of each signal using an nCounter digital analyzer (Nanostring technology, USA), which is used in an embodiment of the present invention, Nikon Eclipse TE2000E (Perfect Focus, 1.4 NA Plan Apo VC 60X oil-immersion lens, Nikon, Japan), which is used in an embodiment of U.S. Pat. No. 8,519,115 and its related published article: Nat. Biotechnol. 2008, 26:317-325, anX-cite 120 metal halide light source (Exfo, USA), automated H117 stage (Prior Scientific, UK), Smart Shutter (Sutter Instrument, USA) or the like, combining each image, and aligning and detecting the signals in a linear combination. U.S. Pat. No. 8,519,115 and its related published article (Nat. Biotechnol. 2008, 26:317-325) are also to be taken as a part of this application.

If the signal-generating region consists of two regions, each of which is labeled with fluorescent dyes capable of generating three different signals (e.g., Alexa Fluor 488 (blue), Cy3 (green) and Cy5 (red)), the linear order of signals creates 9 (=3²) unique signals, thus yielding a total of nine signal-generating regions (or nine different signal probes containing signal-generating regions, each of which produces a different signal), in which each of three signal-generating regions is labeled with the same signal-generating substance (detectable label) and the other six are each labeled with different detectable dyes in a different order. Likewise, as in an embodiment of the present invention, if the signal-generating region consists of six regions, each of which is labeled with fluorescent dyes capable of generating four different signals, there will be, according to the linear order of signals, created 16,384 (=4⁶) unique signals, thus yielding a total of 16,384 signal-generating regions (or 16,384 different signal probes containing signal-generating regions, each of which produces a different signal), and among them, four signal-generating regions are each labeled with the same signal-generating substance (detectable label).

Therefore, in the present invention, when the signal-generating region is designed to contain two or more regions, it may be understood that two or more regions are labeled with the same signal-generating substance or that one or more regions are labeled with a detectable dye producing a different signal from that of the other regions.

The length (the number of nucleotides linked to each other) of each region constituting the signal-generating region may be determined taking into consideration the extent of labeling with the signal-generating substance (that is, the intensity of the signal that is produced). The length of each region may typically range from 0.1 kb (kilobases) to 1.5 kb. In an embodiment of the present invention, nucleic acid amplification is carried out using 100% aminoallyl-dCTP (aminoallyl-dCTP is completely substituted for dCTP) and the other three natural nucleotides (i.e., dATP, dTTP and dGTP), and obtained is a nucleic acid fragment of 903 bp, into which aminoallyl-dCTP is incorporated in one of four nucleotides, and is labeled with a signal-generating substance. If amplification is performed using two types of aminoallyl-NTP and the nucleic acid thus obtained is labeled, it is possible for said region to range from 0.2 kb to 0.5 kb in length. Also, the embodiment of the present invention employs 100% aminoallyl-dCTP, but the use of 30% to 70% aminoallyl-dCTP can provide sufficient dye-coupling to produce sufficient signals in the length of about 0.9 bp.

The signal-generating region is a double-stranded nucleic acid in which two strands are fully or partially double-stranded. As in an embodiment of the present invention, when double-stranded nucleic acid fragments to constitute a signal-generating region are prepared and each labeled and then the labeled double-stranded nucleic acid fragments are linked to each other, for example, by T4 ligase, two single strands of the double-stranded nucleic acid form a fully double-stranded nucleic acid in which the two strands have the same length. In contrast, as in an embodiment of U.S. Pat. No. 8,519,115 (its related published article: Nat. Biotechnol. 2008, 26:317-325), when a single-stranded nucleic acid is made by PCR amplification and isolation, the single-stranded nucleic acid is used as a template with T7 RNA polymerase, T3 polymerase, SP6 polymerase, or the like so as to prepare RNA transcript fragments shorter than the template, and the RNA transcripts are labeled and then complementarily hybridized to the template to constitute a signal-generating region, a partially double-stranded nucleic acid can be formed.

The signal-generating region of the present invention, as described above with regard to the target-molecule-specific binding region, is a double-stranded nucleic acid that may be a nucleic acid analog, such as PNA, and may contain a modified nucleotide.

In the signal probe of the present invention, the target-molecule-specific binding region and the signal-generating region, when both regions are nucleic acids, may be linked to each other using a ligase known in the art, such as T4 ligase. Also, when the target-molecule-specific binding region is an antibody or an aptamer, it may be covalently linked to the signal-generating region by introducing a functional group (a reactive group), such as an amino group, a hydroxyl group, a carboxyl group, a carbonyl group or a thiol group, and inducing amide bonding, ester bonding, thioester bonding, and the like.

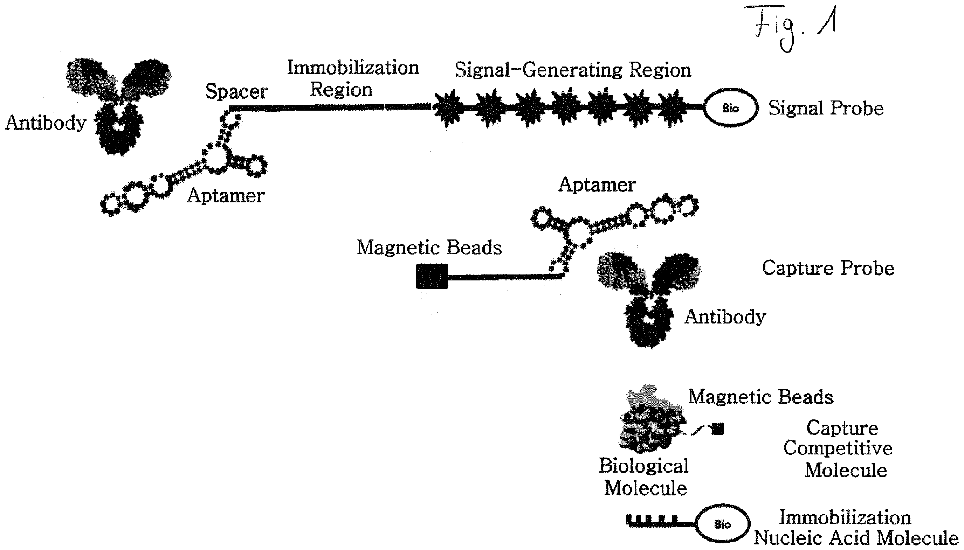

The signal probe, as shown in FIG. 1, may have a spacer and/or an immobilization region between the target-molecule-specific binding region and the signal-generating region. FIG. 1 is a schematic view illustrating the signal probe of the present invention and major components used in the detection method of the present invention for target molecules based on the use of the signal probe.

The spacer is introduced to separate the target-molecule-specific binding region and the signal-generating region. This aims to prevent complexes, formed by the binding of the target-molecule-specific binding region to a target molecule, from acting as obstacles for the detection of signals from the signal-generating part, thereby facilitating the detection of signals from the signal-generating part. The spacer may be a single-stranded nucleic acid, a double-stranded nucleic acid, RNA, DNA, etc., and may contain a nucleic acid analog, such as PNA, or a modified nucleotide. The spacer is not particularly limited in length as long as it facilitates the detection of signals from the signal-generating part. The length may typically range from 20 bp to 50 bp.

The immobilization region is a region to which one or more immobilization nucleic acid molecules (or immobilization molecules), which will be described below, are complementarily bound in order to immobilize and stretch the signal probe of the present invention on an analytical support, as described below, and is composed of a previously known sequence. Thus, the immobilization region is composed of a single-stranded nucleic acid having a sequence complementary to the immobilization nucleic acid molecules, and the previously known sequence may be repeated two or more times in order to allow the binding of two or more immobilization nucleic acid molecules. The immobilization region may be RNA, DNA, or the like, as long as it can bind complementarily to the immobilization nucleic acid molecule, and may also be a nucleic acid analog, such as PNA, or may contain a modified nucleotide. The immobilization region may have a certain length, as long as it can exhibit sufficient binding affinity to the immobilization nucleic acid molecule, and may typically consist of 2 to 20 repetitions of a previously known sequence of 20 to 50 bp in length.

When both the spacer and the immobilization region are present in the signal probe of the present invention, they are covalently linked to an adjacent region, and they can be arranged in a certain order, but it is preferred that the immobilization region be adjacent to the signal-generating region, that is, be present at a 5' end or a 3' end of the signal-generating region. This is because, when the immobilization region further immobilizes the signal probe of the present invention onto an analytical support, the immobilization region should be adjacent to the signal-generating region in order to allow the signal-generating region to be immobilized in a linear or stretched form on the analytical support and to thus facilitate the detection of signals from the signal-generating region.

The signal probe of the present invention may contain a binding tag that is attached to an opposite side of the target-molecule-specific binding region (the opposite side refers to, for example, a 3'-end when the target-molecule-specific binding region is present at a 5'-end).

The binding tag serves to immobilize the signal probe of the present invention on an analytical support, coated with a binding partner capable of binding to the binding tag, through the specific binding to the binding partner. An available representative example of the binding tag is biotin, while the representative binding partner of biotin is streptavidin. Streptavidin is an antibiotic isolated from *Streptomyces avidinii*, belonging to the avidin family, which is a tetrameric protein that can bind to a maximum of four biotin molecules. As the binding tag, as well as biotin, biotin analogs such as desthiobiotin and iminobiotin may be used. As the binding partner coated on the analytical support, as well as streptavidin, streptavidin analogs may be used, which include avidin, traptavidin, NeutrAvidin (ThermoFisher Scientific, USA), and the like.

With respect to the attachment of the binding tag to the signal probe of the present invention, a variety of methods are known in the art, and are described in numerous documents including [Nucleic Acids Res. 1987 Jun 11; 15(11): 4513-4534], [RNA. 2014 Mar; 20(3): 421-427], and [Methods Mol Biol. 2009; 498:185-196], and various kits are commercially available, including products produced by ThermoFisher Scientific and APEXBIO.

In another aspect, the present invention relates to a method of detecting a target molecule in a sample using the aforementioned signal probe.

In detail, the detection method of the present invention comprises the steps of (a) treating a sample intended to be analyzed with a signal probe to form a complex of the signal probe with a target molecule in the sample; and (b) detecting the signal probe of the complex.

In the method of the present invention, the sample to be analyzed may be a certain mixture or solution that contains or is suspected to contain a target molecule intended to be detected and thus needs to be detected. The sample may include biological samples obtained from humans or animals as well as processed samples having an increased concentration of a target molecule through processing of the biological samples. The sample may further include samples containing a target molecule or suspected of containing a target molecule, such as water, food, and industrial waste water, and samples intended to be tested, such as environmental pollutants and toxins. Such samples may contain a suitable diluent and a buffer, and can be a culture of bacteria or viruses, containing a medium or a component thereof, in order to detect the presence of bacteria or viruses.

In the method of the present invention, the sample to be analyzed may preferably be a biological sample taken from humans or animals or a processed sample thereof. The biological sample may be any one of substances taken from humans or animals, which contains or is suspected of containing a target molecule intended to be detected and is thus necessary to test, such as blood, urine, saliva, semen, amniotic fluid, lymphatic fluid, sputum, tissues, etc. The processed sample may be selected from samples having improved protein concentrations using a protein extraction kit, for example, plasma, serum or biological samples, as well as samples having increased concentrations of nucleic acids (DNA or RNA), tissue extracts, cells derived from tissues, cell lysates, cell cultures, and the like.

In the present method, at the step of forming a complex of the signal probe with a target molecule in a sample, when the sample contains a variety of target molecules, it is possible to form multiple different complexes and to detect the complexes using a plurality of signal probes each specific to the target molecules. As described above, when the signal-generating region of the signal probe consists of six regions, each of which is labeled with four different signal-generating substances, it is possible to detect 16,384 (=4⁶) different target molecules.

In the present specification, the term "detection", as used herein, will be understood to refer to qualitative analysis for the presence of a target molecule, and further includes quantitative analysis of the amount and concentration of the target molecule.

The present method, when the target molecule intended to be detected is a protein, may further include, prior to the step (a), the steps of applying the sample onto a nitrocellulose membrane having a high binding affinity to proteins to allow proteins contained in the sample including the target molecule to be adsorbed to the nitrocellulose membrane; and eliminating molecules other than the proteins absorbed to the nitrocellulose membrane, such as lipids, nucleic acids, metabolites, etc., using a suitable washing solution. The washing solution may be used by purchasing any one widely used in the art, or can be manufactured as appropriate, and includes a surfactant and a salt. Examples of surfactants include SDS, Tween 20, Tween 40, Tween 60, Tween 80, Triton X-405, Triton X-100, Tetronic 908, Cholesterol PEG 900, Polyoxyethylene Ether W-1, Span 20, Span 40, Span 85, and mixtures thereof. Examples of salts include acetic salts, lactate, citrate, phosphate, nitrate, sulfate and chloride salts of lithium, sodium, potassium and ammonium, and mixtures thereof (SSC, SSPE, etc.). In particular, as the washing solution can be used TBST solution (10 mM Tris-Cl, pH 8.0, 150 mM NaCl, 0.05% Tween 20), PBST solution (PBS, pH 7.0, 0.05% Tween 20), Tween 20, SSPE solution containing Tween 20 (0.2 M phosphate buffer, 2.98 M NaCl, 20 mM EDTA, pH 7.4), and the like.

After the washing step, the method may further include the step of applying a blocking solution containing a suitable blocking agent, such as bovine serum albumin, to the nitrocellulose membrane to block the protein-unoccupied sites on the membrane surface. This blocking step, when used as the signal probe in which the target-molecule-specific binding region is an antibody in order to form a complex of the signal probe with a target molecule at the next step, serves to prevent the adsorption of the antibody of the signal probe from reducing the detection accuracy. After the blocking step, the signal probe is applied to the membrane in order to perform the step (a) of forming a complex of the signal probe and the target molecule. The blocking step is not essentially required when the target-molecule-specific binding region is not an antibody but an aptamer or the like.

After applying the signal probe, another washing step may be carried out using, for example, the aforementioned washing solution in order to remove non-specifically bound signal probes.

When proteins as the target molecule are detected using the nitrocellulose membrane, after the complex is formed on the nitrocellulose membrane, the detection of the signal probe at the step (b) may be achieved through the steps of (b-1) separating the signal probe from the complex bound to the nitrocellulose membrane and (b-2) detecting the separated signal probe. The separation of the signal probe from the complex is possible by heating the complex-bound nitrocellulose membrane (heating through a suitable buffer or a reaction solution) or by treating with a surfactant during heating.

The signal probe of the present invention, as described above, even when heated, is kept in a stable state without the loss of the signal-generating region, thereby not affecting the accuracy of detection of a target molecule.

The step (b-1) of detecting the separated signal probe may include the steps of (b-1-i) applying the separated signal probe to an analytical support, which is coated with a binding partner to a binding tag of the signal probe, to thus immobilize the signal probe via the binding tag onto the analytical support through the specific interaction between the binding tag and the binding partner; and (b-1-ii) detecting a signal of the signal probe immobilized onto the support.

The signal of the signal probe, which is a signal that is produced from the signal-generating substance labeled to the signal probe, can be detected using a suitable instrument capable of detecting the signal depending on the signal-generating substance that is used. The instrument may be a spectrophotometer, absorption spectrometer, fluorometer, luminometer, chemiluminometer, actinometer, or the like. When the signal is detected using, as the signal-generating substance, Alexa Fluor fluorescent dyes or cyanine fluorescent dyes, it is preferred to use an nCounter digital analyzer (Nanostring technology, USA), which is used in an embodiment of the present invention, a Nikon Eclipse TE2000E (Perfect Focus, 1.4 NA Plan Apo VC 60X oil-immersion lens, Nikon, Japan), which is used in an embodiment of U.S. Pat. No. 8,519,115 and its related published article: Nat. Biotechnol. 2008, 26:317-325, an X-cite 120 metal halide light source (Exfo, USA), an automated H117 stage (Prior Scientific, UK), or a Smart Shutter (Sutter Instrument, USA). The nCounter digital analyzer or the like makes it possible to sense the linear order of a specific signal of the signal-generating region and to count and quantify a target molecule from the sensed signal.

When the linear order of signals is detected using the nCounter digital analyzer or the like, in order to facilitate such signal detection, it is preferred to immobilize the signal-generating region of the signal probe onto an analytical support in a linear or stretched form. This may be achieved by applying an immobilization molecule (or an immobilization nucleic acid molecule) to the signal-probe-immobilized analytical support, wherein the immobilization molecule has a single-stranded nucleic acid sequence complementary to the immobilization region of the signal probe and a binding tag that is linked to the complementary single-stranded nucleic acid sequence and is able to bind to its binding partner coated onto the analytical support. The binding tag of the immobilization molecule may also be biotin or a biotin analog, as described above with regard to the signal probe.

Meanwhile, an analytical support available in the method of this invention is not particularly limited in its form or shape as long as a binding partner (e.g., streptavidin) to the binding tag of the signal probe can be coated onto the surface of the support through covalent or non-covalent bonding.

For instance, the support may be in the form of membranes, beads, microparticles, plates (e.g., glass slides), columns, wells, well plates, and the like, and may be made up of plastic, resin, polysaccharides, silica, functionalized glass, modified silicon, carbon, metals, inorganic glasses, membrane, nylon, natural resin, and the like. The materials for polymeric supports may include polystyrene, polyethylene glycol tetraphthalate, polyvinyl acetate, polyvinyl chloride, polyvinyl pyrrolidone, polyacrylonitrile, polymethyl methacrylate, polytetrafluoroethylene, butyl rubber, styrene butadiene rubber, polyethylene, polypropylene, (poly)tetrafluoroethylene, (poly)vinylidenefluoride, polycarbonate, polymethylpentene, and the like.

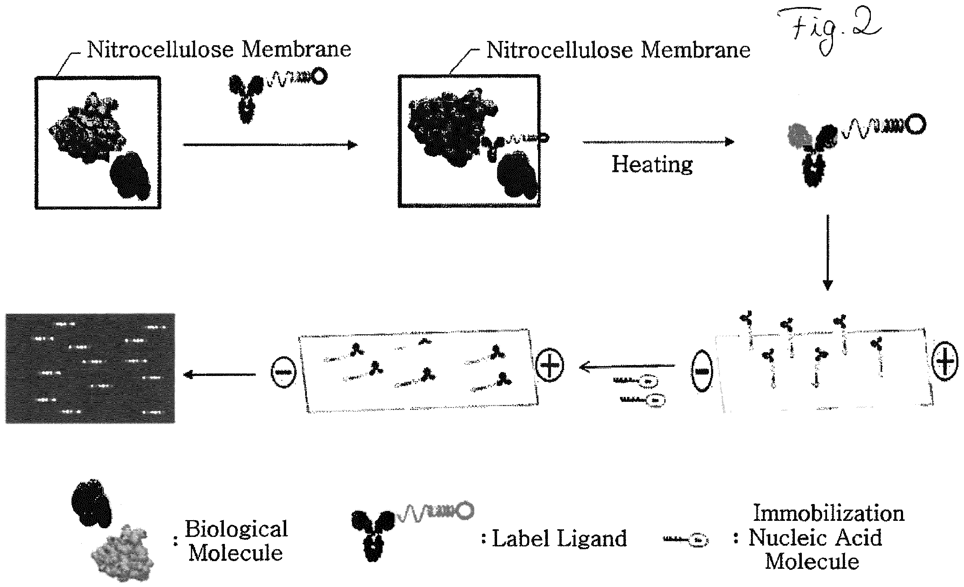

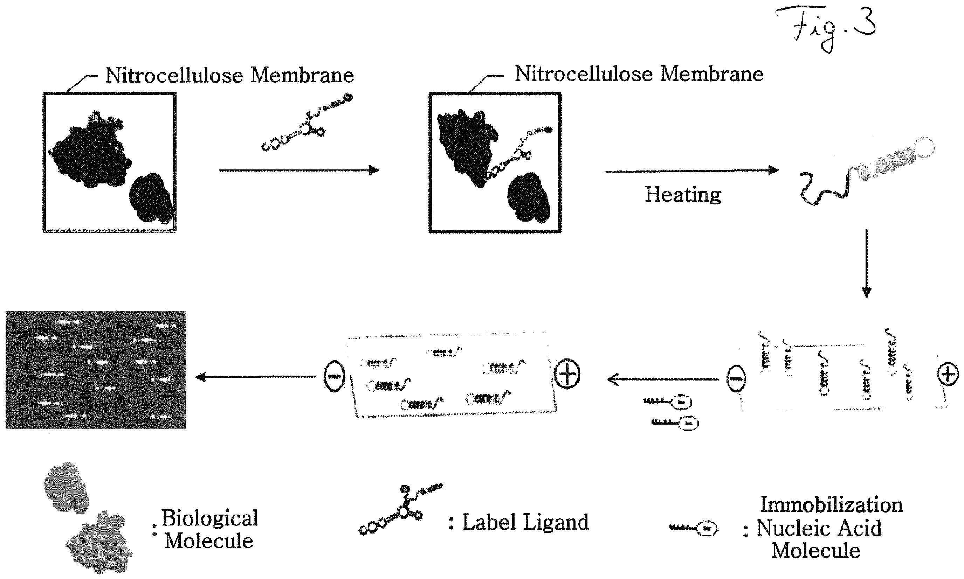

FIG. 1 illustrates the signal probe and major components including the immobilization molecule used in the method of the present invention. FIGS. 2 and 3 show the overall process for detecting a target molecule, a protein, using the aforementioned nitrocellulose membrane, wherein the target-molecule-specific binding region of the signal probe is an antibody or an aptamer, respectively.

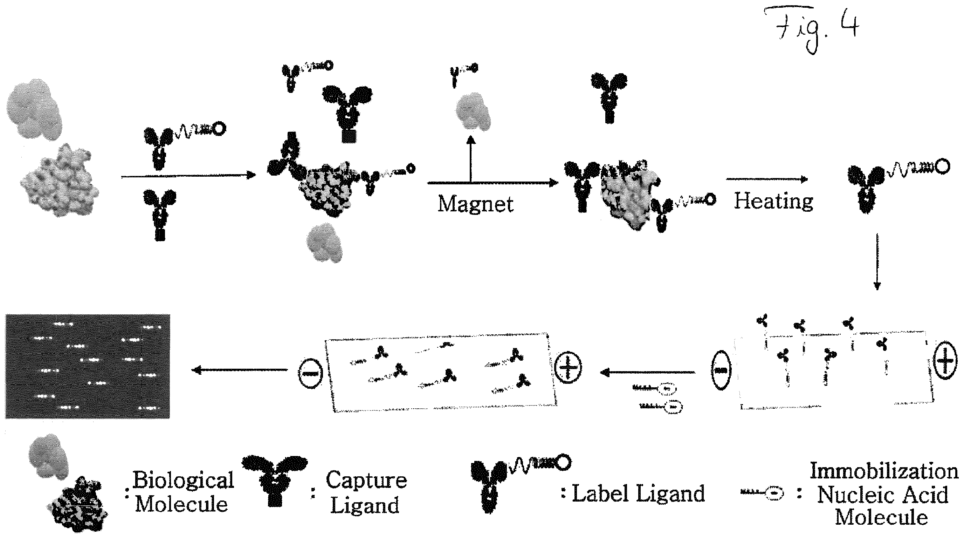

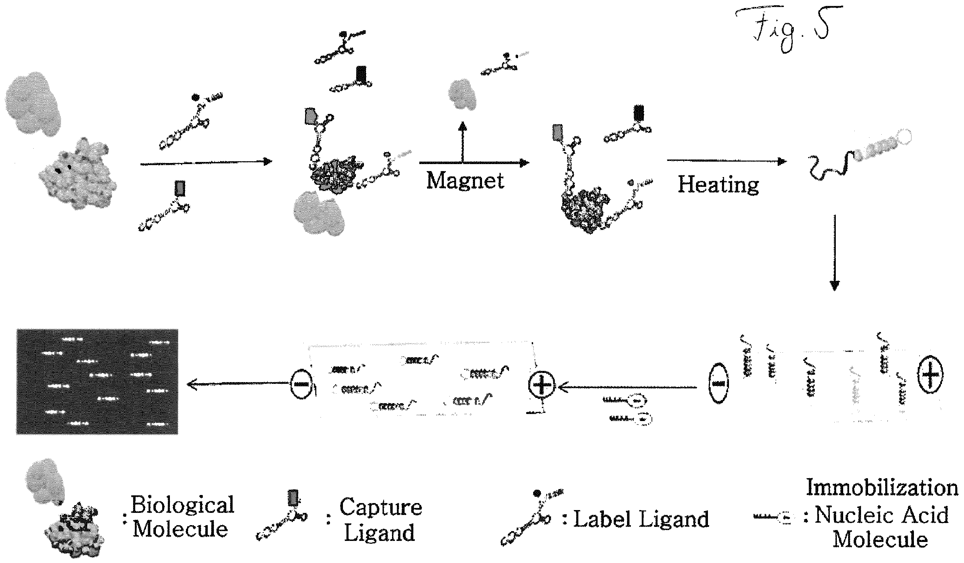

Meanwhile, as shown in FIGS. 4 and 5, the detection method for a target molecule according to the present invention may, in addition to the aforementioned signal probe, further employ a capture probe.

Such a method of the present invention using a capture probe (for convenience, hereinafter referred to as "the capture detection method of the present invention") comprises the steps of (a) treating a sample intended to be analyzed with a signal probe and a capture probe, each of which binds specifically to a target molecule to form a complex of the target molecule, the signal probe and the capture probe, and obtaining a mixture containing the complex as well as a portion of each of the signal probe and the capture probe not participating in the complex formation; and (b) detecting the signal probe of the complex in the mixture.

In the capture detection method of the present invention, the capture probe comprises a region that binds specifically to a different portion from a portion (i.e., an epitope) of a target molecule to which the signal probe specifically binds, wherein a magnetic particle (or a magnetic bead) is connected to the region, as illustrated in FIG. 1.

The magnetic particle has been widely used in the art to isolate proteins, nucleic acids, cells, bacteria, and the like, as disclosed, for example, in U.S. Pat. Nos. 5,665,554, 6,027,945 and 7,078,224, Korean Patent Application Publication No. 2006-0061494, and Korean Pat. No. 0541282, and has a micro- or nano-scale size (i.e. several microns or tens to hundreds of nanometers in size) . The representative magnetic particles that have been employed for isolating biomolecules are iron oxide magnetic particles, including magnetite (Fe₃O₄), maghemite (Fe₂O₃), and hematite (Fe₂O₃). It is necessary that the magnetic particles be modified with non-magnetic materials, such as silica, polymers, gold, and silver, to prevent the interaction and aggregation between magnetic particles in order to be used in the isolation of biomolecules, such as proteins or nucleic acids, and that, in order to allow binding to biomolecules such as proteins or nucleic acids, the magnetic particles be introduced with a functional group, such as carboxyl, epoxy, tosyl, amino, siloxane, etc., or a binding material, such as streptavidin or biotin. Magnetic particles having such properties and manufacturing methods thereof are well known in the art and described in numerous references, including U.S. Pat. Nos. 6,027,945, 6,673,631 and 7,183,002, Japanese Pat. No. 3,253,638, Japanese Patent Application Publication No. 2001-136970, and Korean Patent Application Publication No. 2009-0088299. Also, commercially available are Dynabeads Magnetic Beads (ThermoFisher Scientific, USA), DYNAL M-270 and DYNAL M-280 (Dynal Biotech ASA, Norway), and the like.

In the capture probe of the present invention, a target-molecule-specific binding region may be connected to such a magnetic particle by covalent bonding via a functional group or by non-covalent bonding between biotin and streptavidin. The target-molecule-specific binding region of the capture probe is designed to recognize and bind to a different portion from a portion recognized and bound by a target-molecule-specific binding region of the signal probe so as to prevent competitive binding for the target molecule with the target-molecule-specific binding region of the signal probe.

The target-molecule-specific binding region of the capture probe, like the target-molecule-specific binding region of the signal probe, may be an antibody, an aptamer, a single-stranded nucleic acid, an antigen if the target molecule is an antibody, and the like. If the region is a single-stranded nucleic acid, it may contain a nucleic acid analog, such as PNA or a modified nucleotide.

In the capture detection method of the present invention, the step (a) is performed by treating a sample intended to be analyzed with a signal probe and a capture probe, in which a complex is formed among a target molecule, the signal probe and the capture probe to thus give a mixture containing the resulting complex, a portion of the signal probe not participating in the complex formation, a portion of the capture probe not participating in the complex formation, and non-target molecules.

The next step (b) for the detection of a target molecule serves to detect the signal probe of the complex in the mixture. The detection of the signal probe is based on the capture probe containing magnetic particles, wherein the mixture is applied to a magnet and only the complex containing the capture probe and a portion of the capture probe not participating in the complex formation are adhered to the magnet. During this processing, eliminated are a portion of the signal probe not participating in the complex formation, non-target molecules, and others. During this processing, a washing step using a suitable washing solution may be desirably performed as exemplified above in order to effective removal of the signal probe not participating in the complex formation, non-target molecules and others.

After the complex and others are adhered to the magnet, only the signal probe is separated from the complex. This, as described above, is possible, for example, by heating or by treating with a surfactant with heating. The signal probe, as described above, is maintained in a stable state even under heating or the like because double-stranded nucleic acids constituting the signal probe are cross-linked to each other.

Taking the foregoing into considering, in the capture detection method of the present invention, the step (b) of detecting the signal probe may include the steps of (b-1) applying the mixture to a magnet to adhere only both the complex and a portion of the capture probe not participating in the complex formation to the magnet so as to eliminate other remaining components in the mixture; (b-2) separating the signal probe from the complex adhered to the magnet; and (b-3) detecting the separated signal probe.

The above step (b-3) of detecting only the separated signal probe, as in such a method of detecting a protein using a nitrocellulose membrane as described above, may be achieved by performing the steps of (b-3-i) applying the separated signal probe to an analytic support, which is coated with a binding partner to a binding tag of the signal probe, to immobilize the signal probe via the binding tag onto the analytic support through the specific interaction between the binding tag and the binding partner; and (b-3-ii) detecting a signal of the signal probe immobilized onto the support. For the details of the step (b-3-i) of immobilizing the signal probe onto the analytic support and the detection step (b-3-ii), descriptions with respect to the protein detection method using a nitrocellulose membrane can be referred to in their entireties.

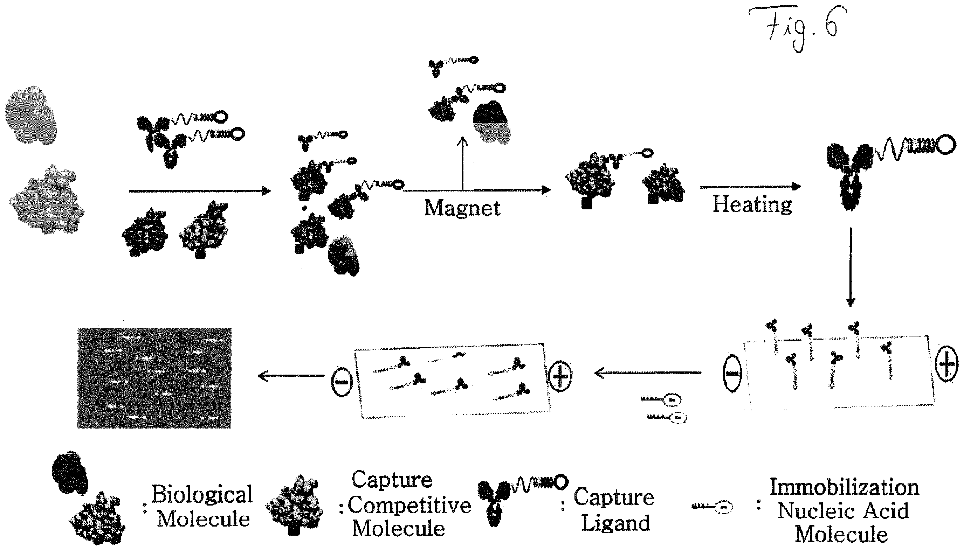

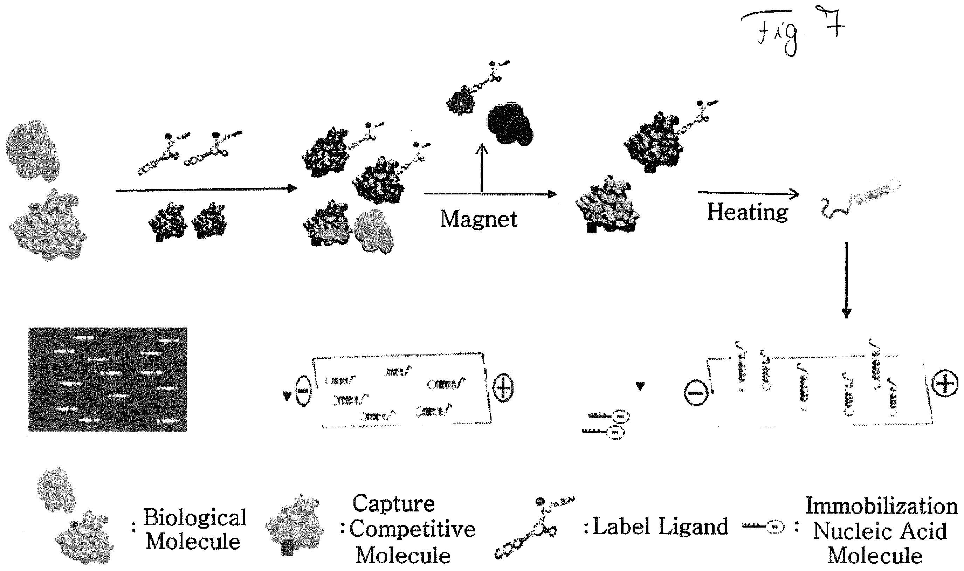

In a further aspect, the present invention, in addition to the signal probe, may further employ a capture competitive molecule as illustrated in FIGS. 6 and 7.

The present method employing the capture competitive molecule (for convenience, hereinafter referred to as "the competitive detection method of the present invention") comprises the steps of (a) treating a sample intended to be analyzed with a signal probe and a capture competitive molecule, each of which binds specifically to a target molecule to form a complex of the capture competitive molecule and the signal probe, and obtaining a mixture containing the complex; and (b) detecting the signal probe of the complex in the mixture.

The mixture contains, in addition to the complex of the capture competitive molecule and the signal probe, a complex of the target molecule and the signal probe, a portion of the signal probe participating in the complex formation, a portion of the competitive molecule participating in the complex formation, a portion of the target molecule participating in the complex formation, and non-target molecules.

In the competitive detection method of the present invention, the capture competitive molecule has a portion the same as the portion (i.e., an epitope) of a target molecule to which the signal probe specifically binds, wherein a magnetic particle is connected to the region. The capture competitive molecule may typically be the same molecule as the target molecule but can be an analogous molecule as long as it can bind to the same portion as a portion (i.e., an epitope) of a target molecule to which the signal probe specifically binds, and can thus compete for binding to the signal probe. Examples of such analogous molecules may include a fusion protein with a protein (Fc of antibody) having a different target protein.

Since the capture competitive molecule has the same binding region for the signal probe as a target molecule, it competes with the target molecule for binding to the signal probe. This allows, when the signal probe is applied in a limited amount to a sample and the capture competitive molecule is used in a predetermined amount, the detection of the capture competitive molecule to provide information about the target molecule in a sample intended to be analyzed (such as the presence or absence of target molecules or the concentrations thereof).

The above-mentioned "the signal probe is applied in a limited amount to a sample", which is intended to mean that the signal probe is used in an amount less than an amount that enables detection both of the capture competitive molecule and the target molecule, theoretically means that it is applied at a concentration allowing the detection of an amount less than the sum of the quantified concentration (quantitative value) of the capture competitive molecule and the estimated concentration (quantitative value) of the target molecule in a sample of interest, but can be applied at a concentration higher than the sum because not all of the applied signal probe binds to the capture competitive molecule or to the target molecule. However, since the concentration of a target molecule in a sample is difficult to estimate, the limited amount of the signal may be generally a concentration that enables detection of less than the quantified concentration of the capture competitive molecule.

The detection and quantification of a target molecule in the sample of interest may be achieved by performing several repeated tests with various concentrations of the signal probe and the capture competitive molecule under the same detection condition, such as using the same reaction solutions, and generating a standard curve that shows the relationship between the concentrations of the applied signal probe and the concentrations of the applied capture competitive molecule. In general, the detected and quantified value of the capture competitive molecule is in inverse proportion to the concentration of the target molecule.

In the competitive detection method of the present invention, the step (b) of detecting the signal probe of the complex in the mixture can be performed in the same way as in the capture detection method.

In detail, the step (b) may include the steps of (b-1) applying the mixture to a magnet to adhere the complex of the capture competitive molecule and the signal probe to the magnet; (b-2) separating only the signal probe from the complex adhered to the magnet; and (b-3) detecting the separated signal probe. At the step (b-1), when the mixture is applied to the magnet, the capture competitive molecule not forming the complex is also adhered to the magnet, but heating makes it possible to separate only the signal probe.

The above step (b-3) of detecting only the separated signal probe, as in the protein detection method using a nitrocellulose membrane and the capture detection method as described above, may be achieved by performing the steps of (b-3-i) applying the separated signal probe to an analytic support coated with a binding partner to a binding tag of the signal probe in order to immobilize the signal probe via the binding tag onto the analytic support through the specific binding between the binding tag and the binding partner; and (b-3-ii) detecting a signal of the signal probe immobilized onto the support. For the details of the step (b-3-i) of immobilizing the signal probe onto the analytic support and the detection step (b-3-ii), descriptions of the protein detection method using a nitrocellulose membrane can be referred to in their entireties.

### Advantageous Effects

As described above, the present invention can provide a stable double-stranded nucleic acid signal probe, in which single-stranded nucleic acids are complementarily joined together to form a double-stranded nucleic acid through cross-linking (or covalent bonding) to thus not be affected by temperature, pH, salinity, ionic strength, or the like.

In addition, in the detection of target molecules, the present invention can provide a method of detecting a target molecule using the stable double-stranded nucleic acid signal probe by forming a complex of the signal probe with the target molecule, separating only the signal probe from the complex by heating or the like, and detecting the signal probe.

### Description of Drawings

FIG. 1 is a schematic view illustrating the signal probe of the present invention and major components used in the detection method of the present invention for target molecules based on the use of the signal probe;
FIGS. 2 and 3 illustrate each step in a method of detecting a protein according to the present invention using a nitrocellulose membrane;
FIGS. 4 and 5 illustrate each step in a method of detecting a target molecule using a capture probe;
FIGS. 6 and 7 illustrate each step in a method of detecting a target molecule using a capture competitive molecule;
FIG. 8 shows a result in which the signal probe of the present invention maintains the double-stranded form of a nucleic acid even at a high temperature;
FIG. 9 shows the result of electrophoresis of a double-stranded nucleic acid fragment or concatenated constructs thereof constituting a signal-generating region of the signal probe according to the present invention;

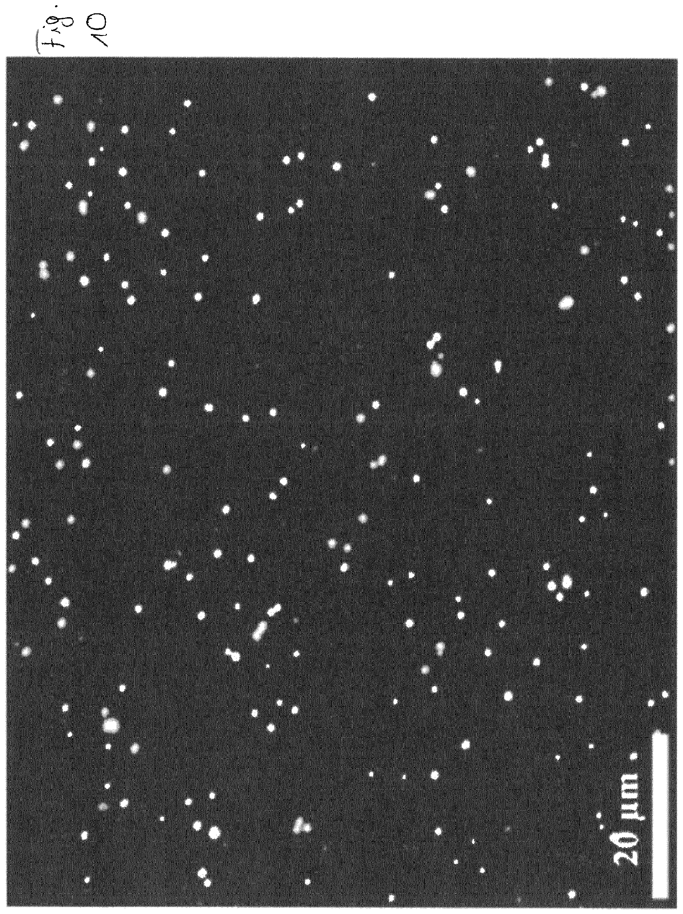

FIG. 10 shows an image obtained using an nCounter digital analyzer (Nanostring technology, USA) for the detection of bacteria using the signal probe of the present invention;
FIG. 11 is a schematic diagram showing results in which the signal probe of the present invention is imaged using an nCounter digital analyzer (Nanostring technology, USA);
FIG. 12 is a graph in which the concentration of a target molecule and the signal intensity are plotted in a log scale versus a log scale after two different standard target molecules are analyzed using the signal probe of the present invention; and
FIG. 13 is a graph in which the concentration of a target molecule and the signal intensity are plotted in a log scale versus a log scale after 13 different target molecules are analyzed using the signal probe of the present invention.

### Mode for Invention

Hereinafter, a better understanding of the present invention may be obtained through the following examples, which are set forth to illustrate but are not to be construed as limiting the present invention.

### Terms used in Examples

The terms used in the following examples have the meanings described below with regard to the terms used in other parts of this specification including the claims.

First, in the following examples, the term "signal probe", as used herein, is used interchangeably with "the first probe", the term "signal-generating region" is used interchangeably with "the color-coded barcode", and the term "target-molecule-specific binding region" of the signal probe is used interchangeably with "the first binding region" or "the first ligand".

In the following examples, the term "capture probe" is used interchangeably with "the second probe" or "the capture probe", and the term "target-molecule-specific binding region" of the capture probe is used interchangeably with "the second binding region" or "the second ligand".

Each region labeled with a signal-generating substance, constituting the signal-generating region of the signal probe, is designated as "a signal tag".

The term "immobilization nucleic acid molecule" is used interchangeably with "the immobilization molecule" in the following examples.

### EXAMPLE 1: Preparation of Reagents

### 1-1. Preparation of the first probe (the signal probe)

The first probe functions to bind to a target molecule and generate a signal unique to a target molecule. The first probe basically consists of a signal-generating region, assigning a unique signal to a target molecule, and the first binding region linked thereto, specifically binding to the target molecule. The first probe may further include an immobilization region and a binding tag.

### 1) Preparation of signal tag

The signal tag indicates the basic unit constituting the signal-generating region and is composed of a double-stranded nucleic acid fragment labeled with a signal-generating substance.

The double-stranded nucleic acid was prepared by performing a polymerase chain reaction (PCR) using M13 DNA as a template and labeling the PCR product of about 900 bp with a signal-generating substance (a detectable label).

The nucleotide sequence of M13 DNA, which is the backbone of the signal tag, was obtained from the GenBank Database system (http://www.ncbi.nlm.nih.gov; Accession No. NC_003287).

PCR was carried out using 100 ng of M13 DNA (NEB, USA) as a template with 0.1 µM of each of a primer pair below (Bioneer, South Korea) and 1.25 units of Taq Polymerase (Promega, USA) according to a standard PCR method, thereby giving a DNA fragment (903 bp).
Forward primer (SEQ ID NO: 1): atcaggcgaatccgttattg
Reverse primer (SEQ ID NO: 2): tcggccttgctggtaatatc

According to the standard method provided by the manufacturer, using as a template the DNA fragment, which is the PCR product thus obtained, another PCR was performed with the above primer pair, aminoallyl-dCTP (TriLink, USA) and three other types of dNTP according to the standard PCR method, thereby obtaining an aminoallyl-dCTP-incorporated DNA fragment.

**[Table 1]**

| The nucleotide sequence of adaptors containing sequences recognized by restriction enzymes | | |
|---|---|---|
| Adaptors | Nucleotide Sequence | SEQ ID NO |
| EcoRI/SmaI Adaptor | 5'-AATTCCCCGGG-3' | SEQ ID NO: 3 |
| | 3'-GGGGCCCp-5' | SEQ ID NO: 4 |
| HindIII/NotI Adaptor | 5'-AGCTTGCGGCCGC-3' | SEQ ID NO: 5 |
| | 3'-ACGCCGGCGp-5' | SEQ ID NO: 6 |
| XhoI/PstI Adaptor | 5'-TCGAGCTGCAGG-3' | SEQ ID NO: 7 |
| | 3'-CGACGTCCp-5' | SEQ ID NO: 8 |
| SalI/BamHI Adaptor | 5'-TCGACGGATCC-3' | SEQ ID NO: 9 |
| | 3'-GCCTAGGp-5' | SEQ ID NO: 10 |

A restriction enzyme map was prepared for the above PCR product to select restriction enzymes not capable of digesting the PCR product, and the adaptors containing recognition sites for the selected restriction enzymes, as listed in Table 1, were purchased (Gene Link, USA).

The adaptors were linked to the aminoallyl-dCTP-incorporated PCR product according to the standard method recommended by the manufacturer to give the combinations described below.
The first domain: 5'-Blunt end-DNA fragment-Eco R1/Sma I adaptor-3'
The second domain: 5'-Eco RI/Sma I adaptor-DNA fragment-Sal I/Bam HIadaptor-3'
The third domain: 5'-Sal I/Bam HI adaptor-DNA fragment-Hind III/Not Iadaptor-3'
The fourth domain: 5'-Hind III/Not I adaptor-DNA fragment-Xho I/Pst1adaptor-3'
The fifth domain: 5'-Xho I/PstI adaptor-DNA fragment-Sal I/Bam HIadaptor-3'
The sixth domain: 5'-Sal I/Bam HI adaptor-DNA fragment-3'

In detail, a ligation reaction was conducted by mixing 3 nmol of the adaptor with 0.3 nmol of the PCR product DNA fragment and adding 1 µl of T4 ligase (5 units/µl; NEB, USA) to 19 µl of the resulting mixture. The products thus ligated were purified to obtain adaptor-attached, aminoallyl-dCTP-incorporated DNA fragments. These DNA fragments were then treated with the above restriction enzymes and purified.

Four different signal-generating substances were prepared, which were N-hydroxysuccinimide (NHS)-ester fluorescent dyes, Alexa 488, Alexa 594, Alexa 647 (Invitrogen, USA) and Cy3 (GE Healthcare, USA).

According to the standard method recommended by the manufacturer, 16-20 µg of the adaptor-attached, aminoallyl-dCTP-incorporated DNA fragment for each domain was dissolved in 20 µl of sterile distilled water (DW) and mixed with 12 µl of a labeling solution (25 mg of NaHCO₃ in 1 ml of sterile DW) to give a reaction solution. Separately, each of the signal-generating substances was dissolved in DMSO at a final concentration of 30 µg/L to give a signal-generating substance solution. Then, the reaction solution was aliquoted into four tubes with 5 µl per tube, and to each tube was added 2 µl of any one of the signal-generating substance solutions, followed by incubation for 1 hr at room temperature in the dark.

After the DNA fragments were labeled with the signal-generating substances in this way, they were purified using the QIAquick PCR Purification Kit (Qiagen, USA), thus giving adaptor-attached DNA fragments, each of which was labeled with the signal-generating substance.

As a result, four types of signal tags were prepared for each domain, which are double-stranded DNA fragments each labeled with any one of the four different signal-generating substances. These signal tags were designated as "H signal tags" for convenience.

2) Induction and confirmation of covalent bonding

1 mg of the H signal tag as prepared above was dissolved in 1 ml of reaction buffer (10 mM Tris (pH 7.0), 200 mM NaCl) and mixed with an equal volume of 0.3 umol of 8-methoxypsoralen (8-MOP; Sigma, USA) dissolved in 95% ethanol, followed by incubation for 1 hr in the dark. Then, this reaction mixture was illuminated with UV light (365 nm, 4 W) for three hours so as to induce covalent bonding between base pairs of the H signal tag chains (A. Arabzadeh et al., International Journal of Pharmaceutics 237 (2002) 47-55).

A four-fold volume of chloroform was added to the resulting reaction mixture to obtain a non-reactive psoralen-removed supernatant through an extraction process. To the supernatant was added 5 M NaCl at a final concentration of 0.2 M and a three-fold volume of ethanol so as to recover the covalent-bond-induced H signal tag. The covalent-bond-induced H signal tag thus obtained was designated a "C signal tag" for convenience.

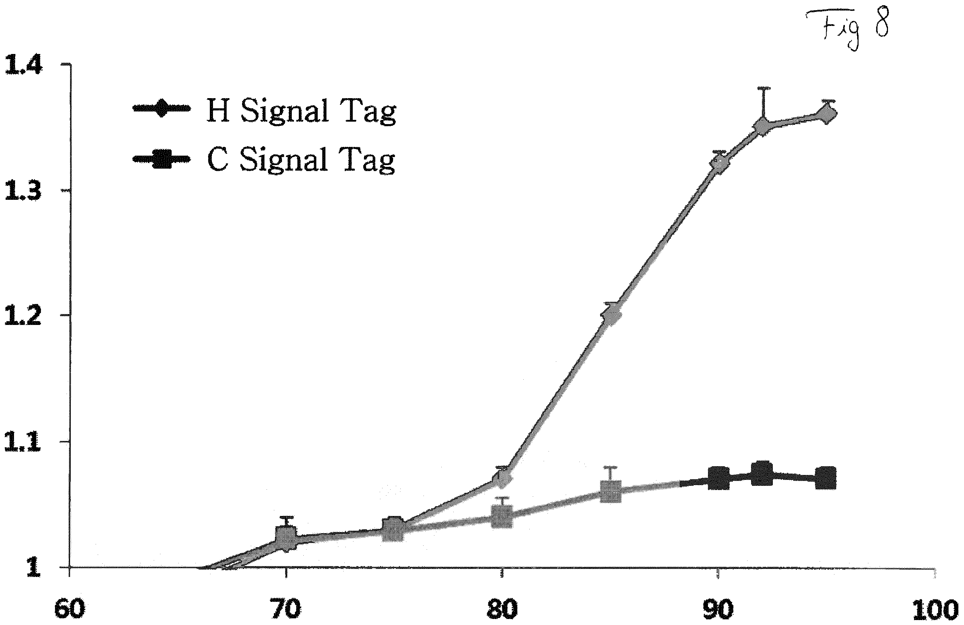

While the H signal tag and its corresponding C signal tag were heated to 95°C at a constant rate of 0.5°C, the absorbance was measured at 260 nm using a UV-1800 spectrophotometer (Shimadzu, Japan). The results are given in FIG. 8.

As the temperature was raised, the double strands of the H signal tag began to dissociate and the duplex was converted to single strands, wherein it was found that the melting temperature (Tm) was about 85°C and the duplex was completely denatured at 95°C to the single-stranded state. In contrast, the C signal tag was found to maintain its double-stranded structure even at 95°C.

### Preparation of the signal-generating region (the color-coded barcode)

The C signal tags thus prepared were used for preparing color-coded barcodes as the signal-generating regions.

First, one signal tag was selected from among the four signal tags for the first domain and placed into each of four tubes at a final concentration of 10 µg/ml. To each tube, each of the four signal tags for the second domain was added at a final concentration of 10 µg/ml. Then, to this mixture was added a reaction solution of T4 ligase (50 mM Tris-HCl, 10 mM MgCl₂, 1 mM ATP, 10 mM DTT, pH 7.5) at a final volume of 100 L. Also, the other three types of signal tags for the first domain were reacted with each signal tag for the second domain according to the same method as described above. Each of the four signal tags for the first domain was mixed with each of the four signal tags for the second domain, and the resulting 16 reaction mixtures were designated as the first-second domain mixture solutions.

Similarly, one signal tag was selected from among the four signal tags for the third domain and placed into each of four tubes at a final concentration of 10 µg/ml. To each tube, each of the four signal tags for the fourth domain was added at a final concentration of 10 µg/ml. Then, to this mixture was added a reaction solution of T4 ligase (50 mM Tris-HCl, 10 mM MgCl₂, 1 mM ATP, 10 mM DTT, pH 7.5) at a final volume of 100 L. Also, the other three types of signal tags for the third domain were reacted with each signal tag for the fourth domain according to the same method as described above. Each of the four signal tags for the third domain was mixed with each of the four signal tags for the fourth domain, and the resulting 16 reaction mixtures were designated as the third-fourth domain mixture solutions.

Similarly, one signal tag was selected from among the four signal tags for the fifth domain and placed into each of four tubes at a final concentration of 10 µg/ml. To each tube, each of the four signal tags for the sixth domain was added at a final concentration of 10 ug/ml. Then, to this mixture was added a reaction solution of T4 ligase (50 mM Tris-HCl, 10 mM MgCl₂, 1 mM ATP, 10 mM DTT, pH 7.5) at a final volume of 100 L. Also, the other three types of signal tags for the fifth domain were reacted with each signal tag for the sixth domain according to the same method as described above. Each of the four signal tags for the fifth domain was mixed with each of the four signal tags for the sixth domain, and the resulting 16 reaction mixtures were designated as the fifth-sixth domain mixture solutions.

Taken together, the above procedures resulted in 16 tubes of the first-second domain mixture solutions, 16 tubes of the third-fourth domain mixture solutions, and 16 tubes of the fifth-sixth domain mixture solutions.

Thereafter, one tube selected from among the 16 tubes of the first-second domain mixture solutions, one tube selected from among the 16 tubes of the third-fourth domain mixture solutions and one tube selected from among the 16 tubes of the fifth-sixth domain mixture solutions were mixed at an equal concentration. To the resulting reaction solution of 95 µl was added 5 µl of T4 ligase (5 units/ µl; NEB, USA). A ligation reaction for 12 hrs resulted in arrangement of the six domain signal tags in a linear, continuous combination, thereby generating a total of 4,096 (16×16×16=4⁶) signal-generating regions as the final constructs, which were designated as "color-coded barcodes" for convenience.

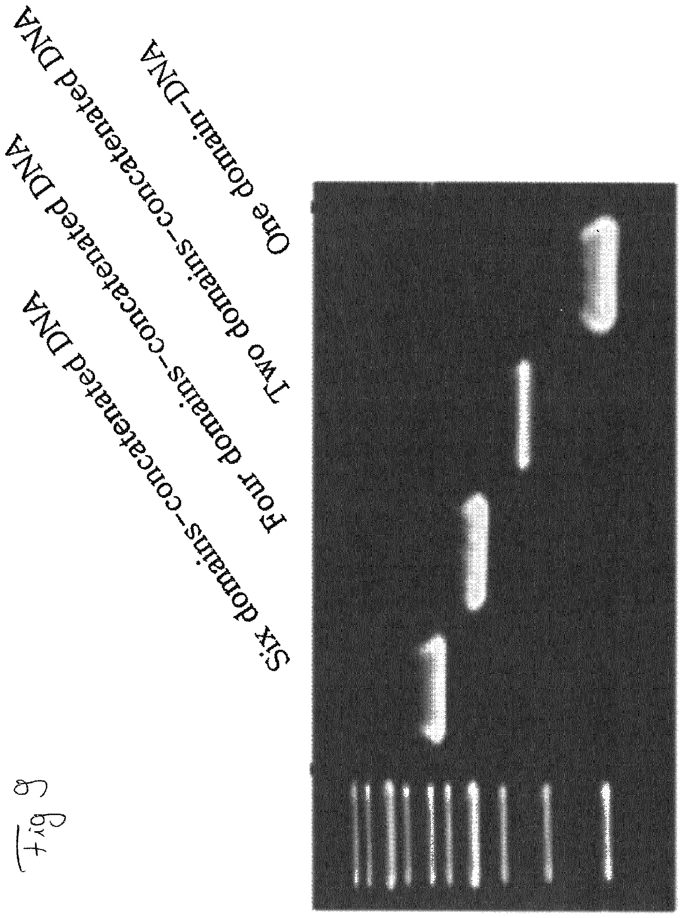

The reaction products at each reaction step were electrophoresed and the result is shown in FIG. 9.

The color-coded barcodes thus obtained have a structure in which the six signal tags (double-stranded nucleic acid fragments labeled with signal-generating substances) are arranged in a linear order and each signal tag is labeled with any one of four different fluorescent dyes (three Alexa dyes and Cy3), whereby the color-coded barcodes generate a unique detectable signal according to the linear combination of signals, and, as will be described below, when linked to the first binding region, that is, the target-molecule-specific binding region, make it possible to distinguish a total of 4,096 (16×16×16=4⁶) target molecules. These color-coded barcodes form a spot of 300 nm in size and are imaged using an epi-fluorescent microscope (US 8,519,115 B2; Nat. Biotechnol. 2008 Mar 26(3) : 293-4).

### 4) Preparation of probes

The first probe is a construct that recognizes and binds to a target molecule and generates a unique signal to detect the target molecule, wherein the target-molecule-specific binding region, that is, the first binding region, is connected with the signal-generating region.

This first probe was prepared in two types, namely a form not immobilized to a support and a form immobilized thereto, depending on the presence or absence of a binding material (e.g., biotin). As the first binding region, used were a single-stranded nucleic acid for nucleic acid detection and an antibody and an aptamer for protein detection.

### (1) The non-immobilizable first probe

A non-immobilizable first probe was designed to have a structure of 5'-color-coded barcode-spacer-the first binding region-3'. The 5' and 3' ends, used here and below, are intended to indicate the direction or order of the arrangement of the nucleic acid when a nucleic acid such as the color-coded barcode or the spacer is used as a constituent factor of the probe.

As the spacer, a single-stranded nucleic acid was synthesized to have the following sequence by a commercial supplier.

Spacer sequence: 5'-AGAAGCGCAGAGCTTGGGCGCAGAACAC-3' (SEQ ID NO: 11)

### ① Preparation of the non-immobilizable first probe containing a single-stranded nucleic acid as the first binding region

A non-immobilizable first probe was prepared using a single-stranded nucleic acid as the first binding region as follows. First, a single-stranded nucleic acid was synthesized to have a construct of 5'-spacer-first binding region-3' (Integrated DNA Technologies, USA). Then, 10 µl of the 5'-spacer-first binding region-3' construct (1 mg/ml) was mixed with 10 µl of a color-coded barcode (1 mg/ml) in a reaction solution (50 mM Tris-HCl, 10 mM MgCl₂, 1 mM ATP, 10 mM DTT, pH 7.5). To the resulting solution of 48 µl was added 2 µl of T4 ligase (5 units/µl; NEB, USA) to allow a ligation reaction, thereby obtaining the non-immobilizable first probe for nucleic acid detection.

### ② Preparation of the non-immobilizable first probe containing an antibody as the first binding region

A non-immobilizable first probe was prepared using an antibody as the first binding region as follows. First, to an antibody was added a reactive group (SH group obtained by reducing disulfide bonds of the antibody using DTT) using the Thunder-Link oligo conjugation system (Innova Biosciences, England) according to the manufacturer's protocol, thereby giving an antibody having a reactive group. In detail, according to the manufacturer's standard method, 100 µl of an antibody (1 mg/ml) was added to the Antibody Activation Reagent vial and incubated for 1 hr at room temperature, thereby generating an activated antibody having a reactive group. Separately, a spacer having a reactive group (NH₂), 5'-spacer-NH₂, was synthesized in the form of a single-stranded nucleic acid (Integrated DNA Technologies, USA). Thereafter, the activated antibody was reacted with the spacer having the reactive group in a solution of N-succinimidyl-S-acetyl-thioacetate to obtain a 5'-spacer-antibody-3' construct. Finally, 10 µl of the 5'-spacer-antibody-3' construct (1 mg/ml) was mixed with 10 µl of a color-coded barcode (1 mg/ml) in a reaction solution (50 mM Tris-HCl, 10 mM MgCl₂, 1 mM ATP, 10 mM DTT, pH 7.5). To the resulting solution of 48 µl was added 2 µl of T4 ligase (5 units/µl; NEB, USA) to induce a ligation reaction, thereby yielding the non-immobilizable first probe for protein detection.

### ③ Preparation of the non-immobilizable first probe containing an aptamer as the first binding region

A non-immobilizable first probe was prepared using an aptamer as the first binding region as follows. First, a construct of 5'-spacer-aptamer-3' was synthesized (Integrated DNA Technologies, USA). Then, 10 µl of the 5'-spacer-aptamer-3' construct (1· mg/ml) was mixed with 10 µl of a color-coded barcode (1 mg/ml) in a reaction solution (50 mM Tris-HCl, 10 mM MgCl₂, 1 mM ATP, 10 mM DTT, pH 7.5). To the resulting solution of 48 µl was added 2 µl of T4 ligase (5 units/µl; NEB, USA) to induce a ligation reaction, thereby yielding the non-immobilizable first probe for nucleic acid detection.

### (2) The immobilizable first probe

An immobilizable first probe was designed to have a construct of 5'-first binding region-spacer-immobilization region-color-coded barcode-biotin-3'. The immobilization region, which consists of repetitions of a previously known sequence, was used to immobilize the first probe onto a support through complementary binding between the immobilization region and an immobilization molecule (that is, an immobilization nucleic acid molecule) containing a sequence complementary to the immobilization region.

The immobilization region was a single-stranded nucleic acid having the following sequence.

The immobilization region: 5'-(GCCACAGCACCTTGGTGCGTAGGATCTG)₁₀-3' (SEQ ID NO: 12)
The number 10, which is an integer, refers to the repeated number of the above sequence.

### ① Preparation of the immobilizable first probe containing a single-stranded nucleic acid as the first binding region

An immobilizable first probe was prepared using a single-stranded nucleic acid as the first binding region as follows. First, a single-stranded nucleic acid was synthesized to have a construct of 5'-spacer-immobilization region-3' (Integrated DNA Technologies, USA). Separately, a color-coded barcode was biotinylated using the Thermo Scientific Biotin 3' End DNA Labeling Kit (Thermo Scientific, USA), thereby generating a 5'-color-coded barcode-biotin-3' construct. In detail, 100 µl of a color-coded barcode (1 mg/ml) was mixed with 5 µl of 5 µM Biotin-11-UTP (Biotin-11-Uridine-5'-triphosphate) in a reaction solution (50 mM Potassium Acetate, 20 mM Tris-acetate, 10 mM Magnesium Acetate, pH 7.9). To the resulting solution of 45 µl was added 5 µl of 1.5 U/µl terminal deoxynucleotidyl transferase (TdT), followed by incubation for 1 hr at 37°C. Then, 10 µl of the 5'-spacer-immobilization region-3' construct (1 mg/ml) was mixed with 10 µl of the 5'-color-coded barcode-biotin-3' construct (1 mg/ml) in a reaction solution (50 mM Potassium Acetate, 20 mM Tris-acetate, 10 mM Magnesium Acetate, pH 7.9). To the resulting solution of 45 µl was added 2 µl of T4 ligase (5 units/µl; NEB, USA) to allow a ligation reaction to generate a 5'-spacer-immobilization region-color-coded barcode-biotin-3' construct, followed by a purification procedure. Finally, 10 µl of the resulting purified construct (1 mg/ml) was mixed with 10 µl of the first binding region (1 mg/ml) in a reaction solution (50 mM Potassium Acetate, 20 mM Tris-acetate, 10 mM Magnesium Acetate, pH 7.9). To the resulting solution of 45 µl was added 2 µl of T4 ligase (5 units/µl; NEB, USA) to allow a ligation reaction, thereby obtaining an immobilizable first probe containing a single-stranded nucleic acid as the first binding region in the construct of 5'-first binding region-spacer-immobilization region-color-coded barcode-biotin-3'.

### ② Preparation of the immobilizable first probe containing an antibody as the first binding region

An immobilizable first probe was prepared using an antibody as the first binding region as follows. First, a 5'-color-coded barcode-biotin-3' construct and an antibody having a reactive group (SH group) were prepared according to the same method as described above, while a single-stranded nucleic acid having a reactive group (NH₂), 5'-NH₂-spacer-immobilization region-3', was synthesized by a commercial supplier (Integrated DNA Technologies, USA). Then, 100 µl of the NH₂-spacer-immobilization region construct (1 mg/ml) was mixed with 100 µl of the 5'-color-coded barcode-biotin-3' construct (1 mg/ml) in a reaction solution (50 mM Potassium Acetate, 20 mM Tris-acetate, 10 mM Magnesium Acetate, pH 7.9). To the resulting solution of 45 µl was added 2 µl of T4 ligase (5 units/µl; NEB, USA) to allow a ligation reaction, thereby obtaining an NH₂-spacer-immobilization region-color-coded-barcode-biotin-3' construct. Thereafter, the antibody having the reactive group was reacted with the above construct for 12 to 24 hrs at room temperature. To the reaction mixture was added the Conjugate Clean Up Reagent (Innova Biosciences, England), and the resulting mixture was allowed to react for 10 min and then centrifuged at 15,000×g for 5 min. This step, the reaction in the Conjugate Clean Up Reagent, followed by centrifugation, was repeated twice to obtain a purified immobilizable first probe containing an antibody as the first binding region in the construct of 5'-first binding region-spacer-immobilization region-color-coded barcode-biotin-3'.

### ③ Preparation of the immobilizable first probe containing an aptamer as the first binding region

An immobilizable first probe was prepared using an aptamer as the first binding region as follows. First, a 5'-color-coded barcode-biotin-3' construct was prepared according to the same method as described above, while a single-stranded nucleic acid, 5'-aptamer-spacer-immobilization region-3', was synthesized by a commercial supplier (Integrated DNA Technologies, USA). Then, 100 µl of the 5'-color-coded barcode-biotin-3' construct (1 mg/ml) was mixed with 100 µl of the 5'-aptamer-spacer-immobilization region-3' construct (1 mg/ml) in a reaction solution (50 mM Potassium Acetate, 20 mM Tris-acetate, 10 mM Magnesium Acetate, pH 7.9). To the resulting solution of 45 µl was added 2 µl of T4 ligase (5 units/µl; NEB, USA) to allow a ligation reaction, thereby obtaining an immobilizable first probe containing an aptamer as the first binding region in the construct of 5'-first binding region-spacer-immobilization region-color-coded-barcode-biotin-3'.

### 1-2. Preparation of the second probe and the capture competitive molecule

The second probe, which is a capture probe, was prepared by binding the second binding region, serving as a target-molecule-specific binding region, to magnetic beads serving as a harvesting material. The competitive molecule was prepared by binding a target molecule (or a target molecule analog capable of competing with the target molecule) to magnetic beads.

### 1) Preparation of the second probe containing a single-stranded nucleic acid or an aptamer as the second binding region

A second probe, containing a single-stranded nucleic acid or an aptamer as the second binding region, was prepared by attaching the second binding region to magnetic beads through biotin-streptavidin interaction. First, a single-stranded nucleic acid or an aptamer, prepared in advance, was biotinylated using the Thermo Scientific Biotin 3 End DNA Labeling Kit (Thermo Scientific, USA) according to the same method as described above in order to obtain 5'-second binding region-biotin-3'. In detail, according to the manufacturer's standard method, 100 µl of the construct (1 mg/ml) was mixed with 5 µl of 5 µM Biotin-11-UTP in a reaction solution (50 mM Potassium Acetate, 20 mM Tris-acetate, 10 mM Magnesium Acetate, pH 7.9). To the resulting solution of 45 µl was added 5 µl of 1.5 U/µl terminal deoxynucleotidyl transferase (TdT), followed by incubation for 1 hr at 37°C. Then, the above-prepared 5'-second binding region-biotin-3' (50 ng/L) was added to an equal volume of the Streptavidin-coupled Dynabeads (5 µg/L) (Dynal Biotech ASA, Norway) and allowed to react by continuous rocking according to the manufacturer's protocol, thereby obtaining a second probe containing a single-stranded nucleic acid as the second binding region.

### 2) Preparation of the second probe containing an antibody as the second binding region

The second probe containing an antibody as the second binding region was prepared by directly binding an antibody to magnetic beads, serving as a harvesting material. First, 100 µg of an antibody was mixed with 5 mg of magnetic beads having a tosyl group (M-280 Tosyl activated magnetic beads, Dynal Biotech ASA, Norway) in a buffer (0.1 M borate buffer, pH 9.5) and incubated for 48 hrs at room temperature. The antibody-bound magnetic beads were separated using a magnet via the tosyl group and washed with the same buffer to remove unbound antibodies. After washing, the antibody-bound magnetic beads (hereinafter, referred to as "capture probe") were reacted with 0.1% bovine serum albumin (BSA) (Sigma, USA) for 4 hrs at 37°C in order to inactivate tosyl groups unbound to molecules. After several washings, the magnetic beads were stored at 4°C in a buffer (0.1 M PBS, pH 7.4).

### 3) Preparation of the capture competitive molecule using a single-stranded nucleic acid as the competitive molecule

The capture competitive molecule using a single-stranded nucleic acid as the competitive molecule was intended to be used for detecting a nucleic acid as a target molecule. This capture competitive molecule was prepared according to the same method as in the preparation of the second probe containing a single-stranded nucleic acid or an aptamer as the second binding region.

### 4) Preparation of the capture competitive molecule using a protein as the competitive molecule

The capture competitive molecule using a protein as the competitive molecule was intended to be used for detecting a protein as a target molecule. This capture competitive molecule was prepared according to the same method as in the preparation of the second probe containing a single-stranded nucleic acid or an aptamer as the second binding region. The same protein as a target protein was used as the competitive molecule.

### 1-3. Preparation of the immobilization molecule

An immobilization molecule was designed to have a nucleotide sequence complementary to the repeated sequence constituting the immobilization region of the first probe, and was prepared by synthesizing a single-stranded nucleic acid biotinylated at its 5' end (Integrated DNA Technologies, USA).

### EXAMPLE 2: Detection of bacteria

Typical food-poisoning bacteria, such as E. *coli, Salmonella, Listeria* and *Staphylococcus,* were detected using the immobilizable first probe, prepared using the antibodies of Table 2 and the aptamers of Table 3 as the first binding region (ligand).

**[TABLE 2]**

| Antibodies binding to surface molecule of food-poisoning bacteria | | |
|---|---|---|
| Antibodies | Catalog No. | Supplier |
| E. coli / Anti-E. coli antibody | ab25823 | Abcam |
| Listeria / Anti-Listeria Antibody | 01-90-95 | KPL |
| Salmonella CSA-1 / Anti-Salmonella CSA-1 Antibody | 01-91-99 | KPL |

**[TABLE 3]**

| Nucleotide sequences of aptamers binding to surface molecule of food-poisoning bacteria | | | |
|---|---|---|---|
| Food Poisoning Bacteria | Nucleotide sequence (5'→3'), SEQ ID NO | | Note |
| *E.coli* | CGCAGUUUGC GCGCGUUCCA AGUUCUCUCA UCACGGAAUA | SEQ ID NO: 13 | Korean Patatent No. 10-0730359 |
| | ACACUGCGUG CUUACGACUU CUGGUCCCAU CAUUCGGCUA | SEQ ID NO: 14 | |
| | AGUUCGAUGA GGGUGACACC GCCAGGAGUG UUUGCUAGAC | SEQ ID NO: 15 | |
| *Salmonella typhimurium* | AGGTCTGTAGGTCTGCGGGGCGTGG | SEQ ID NO: 16 | Korean Patent No. 10-1459547 |
| | CAGCCAGGATGGGAGGTCTGTAGGT CTGCGGGGCGTGG | SEQ ID NO: 17 | |
| *Staphylococcus aureus* | GCAATGGTACGGTACTTCCGGGCTG GCCAGATCAGACCCCGGATGATCAT CCTTGTGAGAACCACAAAGTGCAC GCTACTTTGCTAA | SEQ ID NO: 18 | Aptagen |

As a reaction solution was used 10 mM PBS buffer (137 mM NaCl, 10 mM Phosphate, 2.7 mM KCl, pH 7.4) for the antibody ligand and SELEX buffer (20 mM Tris-Cl, pH 7.6, containing 100 mM NaCl, 2 mM MgCl₂, 5 mM KCl, 1 mM CaCl₂ and 0.02% Tween) for the aptamer ligand. The reaction solution was supplemented with 0.05% to 0.2% (w/v) of sodium azide for suppressing bacterial proliferation and 0.1% to 0.3% (w/v) of bovine serum albumin for inhibiting non-specific binding. The reaction was carried out at 20°C to 30°C.

70 µl of a sample containing bacteria was mixed with 15 µl of the biotinylated first probe and allowed to react for 30 min with rocking. 20 µl of the reaction mixture was applied to a streptavidin-coated glass slide (Nanocs, USA). The glass slide was washed three times with a washing solution containing 0.1X SSPE and 0.1% Tween 20 in order to remove the first probe, which was not bound to bacteria or had non-specific binding. The glass slide was then covered with a coverslip, and spots, generating the signal for the presence of bacteria in a complex formed through the binding of the first probe to bacteria, were imaged using the nCounter digital analyzer (Nanostring technology, USA), and the result is given in FIG. 9.

### EXAMPLE 3: Detection of nucleic acid and protein

### 3-1. Nucleic acid detection

The nucleic acid detection was conducted with an immobilizable first probe, containing a single-stranded nucleic acid of Table 4 below as the first binding region, and a second probe, containing a single-stranded nucleic acid of Table 4 below as the second binding region, using β-actin, IL17 and IL17RA mRNA molecules as a target molecule. The single-stranded nucleic acids, listed in Table 4 below, for β-actin, IL17 and IL17RA mRNA molecules were synthesized (Bioneer, South Korea) and used in the preparation of the first probe and the second probe. The nucleotide sequences of the target molecules, β-actin, IL17 and IL17RA mRNA, were obtained from the GenBank database system (http:// www.ncbi.nlm.nih.gov) (IL17: Accession number NM_002190; and IL17RA: Accession number NM_014339).

**[TABLE 4]**

| Sequences of binding regions for target mRNAs | | |
|---|---|---|
| Types | The first binding region (5'-3') | The second binding region (5'-3') |
| β-actin mRNA | Gggcgacgaggcccagagcaagaga (SEQ ID NO: 19) | Ggcatcctcaccctgaagtacccca (SEQ ID NO: 20) |
| IL17 mRNA | Ccctcaggaaccctcatccttcaaa (SEQ ID NO: 21) | gacagcctcatttcggactaaactc (SEQ ID NO: 22) |
| IL17RA mRNA | Ggagcagaagcctcccagccactag (SEQ ID NO: 23) | Ccttttgggctcagtctctccaata (SEQ ID NO: 24) |

Human cartilage tissue (Promocell, Germany) was used as a sample to be analyzed. First, mRNA, as the target molecule, was isolated from the sample using the AllPrep DNA/RNA/Protein Mini kit (Qiagen, USA). In detail, according to the manufacturer's protocol, the cartilage tissue was dissolved in a buffer provided by the manufacturer, loaded onto a column and centrifuged to bind RNA to the column membrane. The RNA bound to the column membrane was recovered by washing the column, eluting RNA from the column membrane and centrifuging the column.

The isolated RNA (100 ng/µl) was mixed with the first probe (200 ng/µl) and the second probe (200 ng/µl) with rocking to form a complex of the first probe, the target mRNA and the second probe through hybridization. Hybridization was carried out at 65°C in a reaction solution containing 0.5 M NaHPO₄, 7% sodium dodecyl sulfate (SDS) and 1 mM EDTA. Then, the reaction mixture was applied to a magnet to immobilize the complex onto the magnet through the magnetic beads of the second probe, followed by washing with a washing solution in order to remove the unbound first probe and non-target RNA. The washing was performed three times: once with 6X SSC (standard saline citrate)/0.1% SDS at 8°C, once with 0.1X SSC/0.1% SDS at 68°C, and once 0.2X SSC/0.1% SDS at 42°C.

The magnet, to which the complex of the first probe, the target mRNA and the second probe was bound, was incubated in a solution containing 0.1X SSPE and 0.1% Tween 20 for 5 min at 95°C in order to separate the first probe from the complex, thereby obtaining a supernatant containing the first probe.

After the supernatant was obtained, the first probe was immobilized onto a streptavidin-coated coverslip using the nCounter digital analyzer (Nanostring technology, USA), which enables molecular counting, according to the manufacturer's protocol, and the immobilized spots were imaged to thus be detected and quantified.

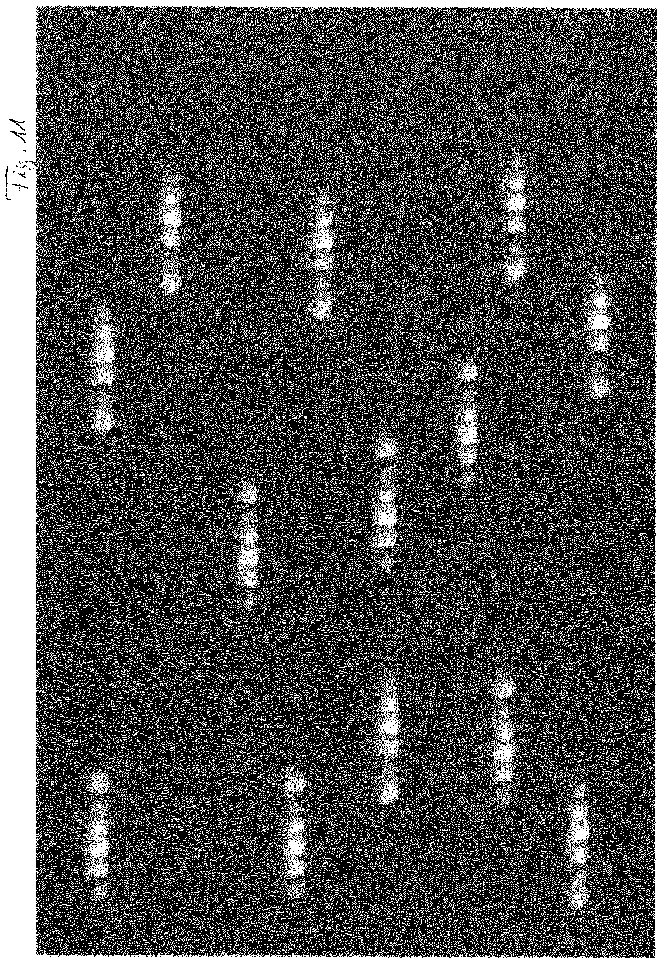

In detail, 3 µl of the above-obtained supernatant was mixed with 1 µl of a 1:5,000 dilution of 0.1% TetraSpeck Fluorescent Microspheres, loaded into a fluid device in the above analyzer equipped with a streptavidin-coated coverslip (Optichem, Accelr8 Technology Corporation) in order to induce attachment of the first probe onto the coverslip as a support, through the interaction between biotin in the first probe and streptavidin in the coverslip. Thereafter, the surface was washed once with 90 µl of 1X TAE and then treated with 40 µl of 1X TAE to stretch the first probe, followed by applying 160 V/cm for 1 min. To the stretched first probe was added 60 µl of a 500 nM immobilization molecule solution in order to induce complementary binding to an immobilization region, consisting of a repeated sequence, of the first probe, and to thus further immobilize the first probe onto the surface. During the immobilization process, the voltage was maintained for 5 min. After the immobilization process, the TAE solution was removed, and an anti-photobleaching agent for photography, SlowFade reagent (Invitrogen), was added for imaging. Thereafter, four images were obtained at four different excitation wavelengths (480, 545, 580 and 622) corresponding to the four different fluorescent dyes through a camera of the above analyzer. The obtained four images were integrated (spatial clustering), and an image for the first probe was obtained as shown in the schematic diagram of FIG. 11. During imaging, the resolution of the above analyzer was set to 600 FOV (field of view). Data were downloaded from the above analyzer and analyzed in Excel according to the manufacturer's instructions. Data were normalized for a standard material and a target molecule in a sample using an initially provided standard curve of a positive test control. The normalized values were used for overall average normalization of target molecule values using the values of the reference materials contained in a sample. The quantitative values of target molecules were calculated by averaging the analysis results of three repetitions.

Quantitative results were obtained as described above using the above analyzer through molecular counting for the target molecule mRNA. The results thus obtained were found to be similar to the RT-PCR result for the target mRNA.

### 3-2. Protein Detection

### 3-2-1. Protein detection using only the first probe

The target molecules of Table 5 below were detected using only an immobilizable first probe containing a polyclonal antibody as listed in Table 5 below as the first binding region for a target molecule. The overall process of protein detection can be seen in FIGS. 2 and 3.

**[TABLE 5]**

| Target molecules and antibodies thereto | | |
|---|---|---|
| Target molecule/antibody | Catalog No. | Supplier |
| NGF/NGF Anti-NGF/NGFβ antibody | OKBB00229 | Aviva Systems Biology |
| Amyloid beta (A4) Precursor Protein (APP) Anti-Amyloid beta (A4) Precursor Protein (APP) antibody | ab12266 | Abcam |
| (Tau) / Anti- (Tau) antibody | T6402 | SIGMA |
| phospho-Tau (pSer400) / Anti-phospho-Tau (pSer400) antibody | T1700 | SIGMA |
| C Reactive Protein (CRP)/ Anti-C Reactive Protein (CRP)antibody | ab99995 | abcam |
| TGF alpha / Anti-TGF alpha antibody | ab9585 | abcam |
| IL1 beta / Anti-ILl beta antibody | ab2105 | abcam |
| Serum Amyloid A / Anti-Serum Amyloid A antibody | ab200584 | abcam |
| p53 / p53 Antibody (DO-7) | MA5-12557 | abcam |
| CEACAM5 / CEACAM5 Polyclonal Antibody | MBS170144 | BioSource |
| alpha Fetoprotein / alpha Fetoprotein antibody (alpha-Fetoprotein) (N-Term) | ABIN356914 | Biocompare |
| E. coli Enoyl-ACP Reductase/ Anti-E. coli Enoyl-ACP Reductase antibody | | Sino Biological Inc., China |
| Phototropin 1 / Anti-Phototropin 1 antibody | | Cosmo Bio Co. Ltd., Japan |

First, each target molecule of Table 5 was added at a concentration of 100 µg/ml to 10 mM PBS buffer (137 mM NaCl, 10 mM Phosphate, 2.7 mM KCl pH 7.4) and serially diluted to give final concentrations of 1,000 pg/ml, 100 pg/ml, 10 pg/ml, 0.1 pg/ml and 0 pg/ml. The resulting serial dilutions for the thirteen biological molecules were mixed at various concentrations to give mixture samples to be analyzed.

In addition, the first probe was added at a concentration of 200 ng/ml to 10 mM PBS buffer (137 mM NaCl, 10 mM Phosphate, 2.7 mM KCl pH 7.4) to give an immobilizable first probe solution.

A nitrocellulose membrane disc was added to the reaction solution containing the sample to be analyzed and allowed to react for 30 min with rocking to attach proteins in the sample onto the membrane. Then, the disc was washed with 150 µl of 0.1X SSPE/0.1% Tween 20 and allowed to react in a blocking solution (2% (w/v) bovine serum albumin (BSA)). 70 µl of the immobilizable first probe solution for each target molecule was added and the solution was rocked for 1 hr to induce the formation of a complex between the target molecule and the first probe. The disc was then washed with 150 µl of 0.1X SSPE/0.1% Tween 20 three times so as to eliminate excess first probes, not forming the complex, and the first probe forming a non-specific complex.

In order to separate and harvest the first probe from the washed disc, the washed disc was heated in 0.1X SSPE/0.1% Tween 20 for 5 min at 95°C to thus induce separation of the first probe from the complex, thereby obtaining a supernatant containing the first probe.

After the supernatant was obtained, according to the same method as in Example 3-1, the first probe was immobilized onto a streptavidin-coated coverslip using the nCounter digital analyzer (Nanostring technology, USA), which enables molecular counting, according to the manufacturer's protocol, and the immobilized spots were imaged to thus be detected and quantified.

### 3-2-2. Protein detection using the first probe and the second probe (capture detection reaction)

In the same way as in the immobilizable first probe containing the polyclonal antibody of Table 5 as the first binding region for a target molecule, the target molecules of Table 5 were detected using a second probe containing a polyclonal antibody as listed in Table 5 as the second binding region for a target molecule. The overall process of protein detection can be seen in FIGS. 4 and 5.

A sample to be analyzed was prepared according to the same method as in the above Example 3-1. 25 µl of the sample to be analyzed was mixed with 70 µl of an immobilizable first probe solution for each target molecule and 70 µl of a second probe and rocked for 1 hr to induce the formation of a complex of the first probe, the target molecule and the second probe. Then, the reaction mixture was applied to a magnet to attach the complex onto the magnet through the magnetic beads of the second probe. The beads bound to the magnet were then washed with a washing solution so as to eliminate unbound first probes and non-target molecules. The washing was carried out three times using 150 µl of 0.1X SSPE/0.1% Tween 20.

Then, the complex-adhered magnet was heated in 0.1X SSPE/0.1% Tween 20 for 5 min at 95°C in order to separate the first probe from the complex, thereby obtaining a supernatant containing the first probe.

After the supernatant was obtained, according to the same method as in Example 3-1, the first probe was immobilized onto a streptavidin-coated coverslip using the nCounter digital analyzer (Nanostring technology, USA), which enables molecular counting, according to the manufacturer's protocol, and the immobilized spots were imaged to thus be detected and quantified.

The quantified results are given in Table 6 below.

**[TABLE 6]**

| The actual and analyzed concentrations of target molecules in the prepared mixture samples | | | |
|---|---|---|---|
| Target molecules | Serial No. | Actually used amount (pg/mL) | Measured amount (pg/mL) |
| NGF/NGF | 1 | 12.0 | 12.5 |
| Amyloid beta (A4) Precursor Protein (APP) | 2 | 1.0 | 0.8 |
| Tau | 3 | 1.0 | 0.8 |
| phospho-Tau (pSer400) | 4 | 0.2 | 0.2 |
| C Reactive Protein (CRP) | 5 | 130 | 125.5 |
| TGF alpha | 6 | 6.0 | 6.2 |
| IL1 beta / Anti-IL1 beta antibody | 7 | 40.0 | 38.1 |
| Serum Amyloid A | 8 | 5.0 | 5.0 |
| p53 | 9 | 2.5 | 2.5 |
| CEACAM5 | 10 | 2.0 | 1.9 |
| alpha Fetoprotein | 11 | 0.5 | 0.6 |
| E. coli Enoyl-ACP Reductase | 12 | 80.0 | 81.9 |
| Phototropin 1 | 13 | 10.0 | 9.5 |

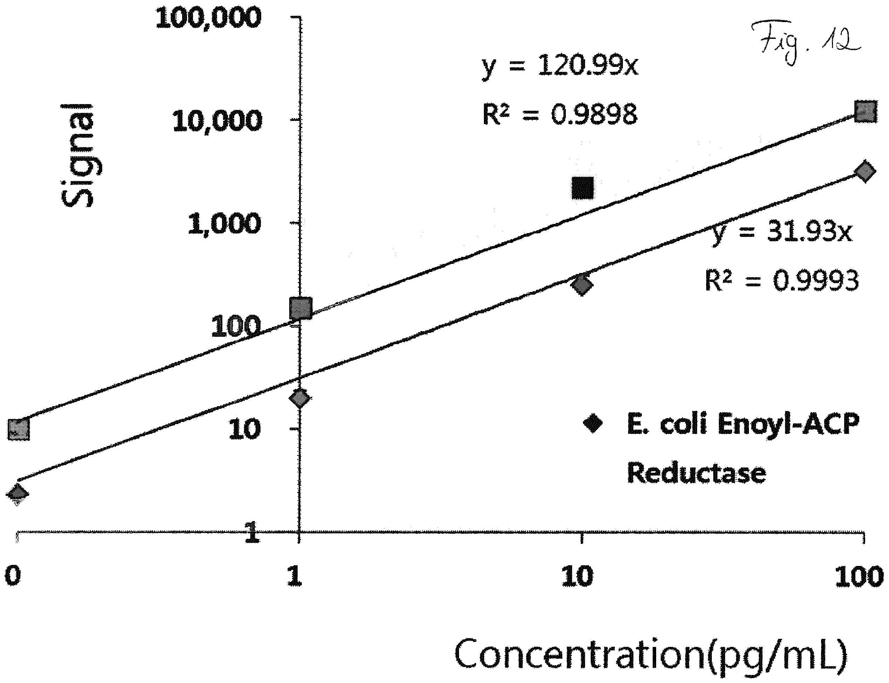

Analysis was performed three time for a mock comparative group, consisting of two standard materials (E. coli Enoyl-ACP Reductase and Phototropin 1), and a control group containing a standard material and other target molecules. The mock comparative group consisting of the standard materials was used to generate a standard concentration curve and normalizing data having a difference in reaction, purification and collection efficiencies. The linearity, dynamic range and reproducibility of the standard materials were examined and are shown in FIG. 12. FIG. 12 shows the results (N=6) obtained through measurement of the standard material of the mock comparative group. As shown by the overlapping spots on the log-log plot, the reproducibility of the values (counts) of control signals for each analysis was found to be very high, in the range from 0.1 pg/ml to 100 pg/ml. Also, as a result, the linear regression correlation coefficient of concentration versus count was linear with a change of from 0.1 log to ≥0.989 log of concentration.

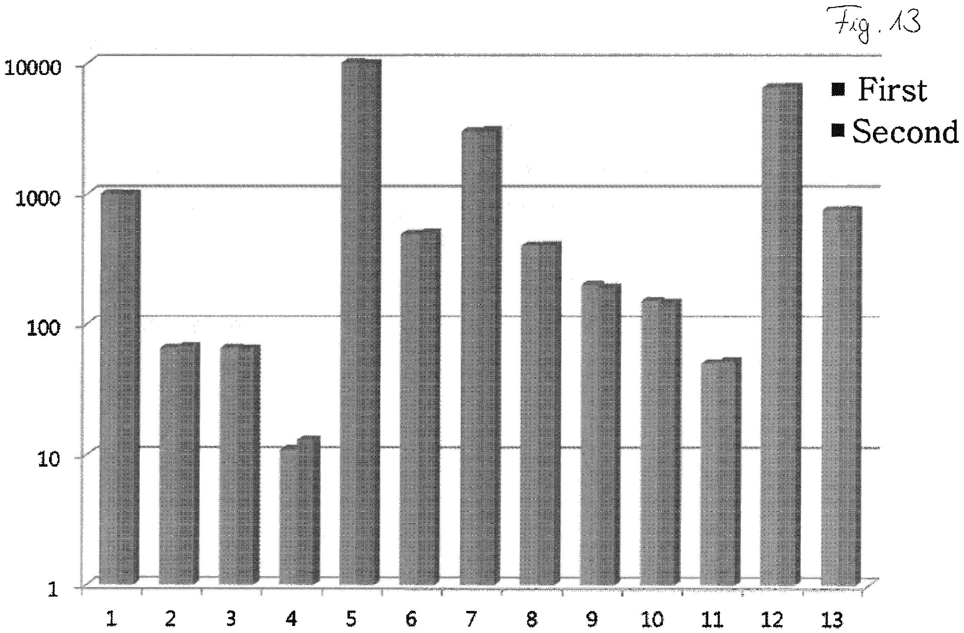

The thirteen biological molecules of Table 6 were detected in two independent reactions, and the normalized results are shown in a log-log scale in FIG. 13, in which the deviation for each signal ranged from 0.7 to 35.4.

### 3-2-3. Protein detection using the first probe and the capture competitive molecule (competitive detection reaction)

The target molecules of Table 5 were detected using a first probe containing an antibody listed in Table 6 as the second binding region and a capture competitive molecule using each protein of Table 5. The overall process of protein detection can be seen in FIGS. 6 and 7.

A sample to be analyzed was prepared according to the same method as in the above Example 3-1. 25 µl of the sample to be analyzed was mixed with 70 µl of an immobilizable first probe solution for each target molecule and 70 µl of a capture competitive molecule (30 µg/µl) and rocked for 1 hr to induce the formation of a complex of the first probe, the target molecule and the capture competitive molecule. Then, the reaction mixture was applied to a magnet to attach the complex onto the magnet through the magnetic beads of the capture competitive molecule. The bead bound to the magnet was then washed with a washing solution so as to eliminate unbound first probes, non-target molecules and the first probe-target molecule complex, thereby harvesting only the complex between the first probe and the capture competitive molecule. The washing was carried out three times using 150 µl of 0.1X SSPE/0.1% Tween 20.

Then, the magnet, to which the complex of the first probe and the capture competitive molecule was adhered, was heated in 0.1X SSPE/0.1% Tween 20 for 5 min at 95°C in order to separate the first probe from the complex, thereby obtaining a supernatant containing the first probe.

After the supernatant was obtained, according to the same method as in Example 3-1, the first probe was immobilized onto a streptavidin-coated coverslip using the nCounter digital analyzer (Nanostring technology, USA), which enables molecular counting, according to the manufacturer's protocol, and the immobilized spots were imaged to thus detect and quantify the same. A standard curve was generated from the quantitative results of the capture competitive molecule, and quantitative results of target molecules were obtained from the standard curve.

## Claims

1. A double-stranded nucleic acid signal probe, which is capable of generating a unique signal to a target molecule, comprising:
(i) a region that binds specifically to the target molecule; and
(ii) a signal-generating region that is linked to the target-molecule-specific binding region,
wherein the signal-generating region is a double-stranded nucleic acid formed via complementary crosslinking, and the double-stranded nucleic acid contains two or more regions, each of which is labelled with a same or different signal-generating substance.

2. The signal probe of claim 1, wherein one or more of the two or more regions generate a signal different from those of other remaining regions.

3. The signal probe of claim 1, wherein the signal-generating region is a double-stranded nucleic acid in which two strands are fully or partially double-stranded.

4. The signal probe of claim 1, wherein the target-molecule-specific binding region is a single-stranded nucleic acid, an antibody, an antigen, or an aptamer.

5. The signal probe of claim 1, wherein the signal-generating substance is two or more fluorescent materials each having a different color.

6. The signal probe of claim 1, wherein an immobilization region, which is a previously known sequence, is connected adjacent to the signal-generating region.

7. The signal probe of claim 1, wherein an immobilization region, which is a previously known sequence, is further connected adjacent to the signal-generating region and the previously known sequence is present in two repetitions.

8. The signal probe of claim 1, wherein a spacer is linked between the target-molecule-specific binding region and the signal-generating region.

9. The signal probe of claim 1, wherein a binding tag is attached to an opposite side of the target-molecule-specific binding region.

10. The signal probe of claim 1, wherein a binding tag is attached to an opposite side of the target-molecule-specific binding region and the binding tag is biotin or an analog thereof.

11. A method of detecting a target molecule in a sample intended to be analyzed, comprising the steps of:
(a) treating the sample intended to be analyzed with a signal probe having a target-molecule-specific binding region to form a complex of the signal probe and the target molecule in the sample; and
(b) detecting the signal probe of the complex,
wherein the signal probe comprises:
(i) the region that binds specifically to the target molecule; and
(ii) a signal-generating region that is linked to the target-molecule-specific binding region,
wherein the signal-generating region is a double-stranded nucleic acid formed via complementary crosslinking, and the double-stranded nucleic acid contains two or more regions, each of which is labelled with a same or different signal-generating substance,
and the step of detecting the signal probe is performed by detecting a signal of the signal-generating region.

12. The detection method of claim 11, wherein the target-molecule-specific binding region is a single-stranded nucleic acid, an antibody, an antigen, or an aptamer.

13. The detection method of claim 11, wherein the step of detecting the signal probe comprises the steps of (i) separating the signal probe from the complex; and (ii) detecting the signal of the separated signal probe.

14. A method of detecting a target protein in a sample intended to be analyzed, comprising the steps of:
(a) applying the sample intended to be analyzed to a nitrocellulose membrane to adhere proteins in the sample onto the nitrocellulose membrane;
(b) treating the protein-adhered nitrocellulose membrane with a blocking agent to block a protein-non-adhered surface of the nitrocellulose membrane;
(c) applying a signal probe having a target protein-specific binding region to the blocked nitrocellulose membrane to form a complex of the signal probe and the target protein in the sample; and
(d) detecting the signal probe of the complex,
wherein the signal probe comprises:
(i) the region that binds specifically to the target protein; and
(ii) a signal-generating region that is linked to the target protein-specific binding region,
wherein the signal-generating region is a double-stranded nucleic acid formed via complementary crosslinking, and the double-stranded nucleic acid contains two or more regions, each of which is labelled with a same or different signal-generating substance,
and the step of detecting the signal probe is performed by detecting a signal of the signal-generating region.

15. The detection method of claim 14, wherein the target-molecule-specific binding region is an antibody or an antigen.

16. The detection method of claim 14, further comprising a washing step after the step (a) in order to eliminate molecules other than the protein adhered onto the nitrocellulose membrane.

17. The detection method of claim 14, further comprising a washing step after the step (c) in order to eliminate non-specific bound molecules.

18. The detection method of claim 14, wherein the step (d) of detecting the signal probe comprises steps of (d-1) separating the signal probe from the complex; and (d-2) detecting the separated signal probe.

19. The detection method of claim 14, wherein a binding tag is attached to an opposite side of the target-molecule-specific binding region, and
the step (d) of detecting the signal probe comprises steps of (d-1) separating the signal probe from the complex; and (d-2) detecting the separated signal probe,
wherein the step (d-2) of detecting the separated signal probe comprises the steps of:
(d-2-i) applying the separated signal probe to an analytical support, which is coated with a binding partner to the binding tag, to thus immobilize the signal probe via the binding tag onto the analytical support through specific interaction between the binding tag and the binding partner; and
(d-2-ii) detecting a signal of the signal probe immobilized onto the support.

20. The detection method of claim 19, wherein the binding tag is biotin or an analog thereof.

21. The detection method of claim 19, wherein the binding partner is streptavidin or an analog thereof.

22. The detection method of claim 19, wherein an immobilization region, which is a previously known sequence, is further connected adjacent to the signal-generating region, and
the step (d-2-ii) of detecting the signal of the signal probe comprises the steps of:
applying, to the support, an immobilization nucleic acid molecule having a single-stranded nucleic acid sequence complementary to the immobilization region and a binding tag that is linked to the sequence and is able to specifically bind to the binding partner; and
detecting the signal of the signal probe.

23. The detection method of claim 22, wherein the binding tag of the immobilization nucleic acid molecule is biotin or an analog thereof.

24. A method of detecting a target protein in a sample intended to be analyzed, comprising the steps of:
(a) treating the sample intended to be analyzed with a signal probe, binding specifically to a target molecule, and a capture probe, containing a region that binds specifically to a portion of a target molecule different from a portion to which the signal probe specifically binds, and a magnetic particle that is connected to the region to thus form a complex of the target molecule, the signal probe and the capture probe, and obtaining a mixture containing the complex as well as a portion of each of the signal probe and the capture probe not participating in the complex formation; and
(b) detecting the signal probe of the complex in the mixture,
wherein the signal probe comprises:
(i) the region that binds specifically to the target protein; and
(ii) a signal-generating region that is linked to the target protein-specific binding region,
wherein the signal-generating region is a double-stranded nucleic acid formed via complementary crosslinking, and the double-stranded nucleic acid contains two or more regions, each of which is labelled with a same or different signal-generating substance, and
the step of detecting the signal probe is performed by detecting a signal of the signal-generating region.

25. The detection method of claim 24, wherein the target-molecule-specific binding region is a single-stranded nucleic acid, an antibody, an antigen, or an aptamer.

26. The detection method of claim 24, wherein the step (b) of detecting the signal probe comprises steps of:
(b-1) separating only the signal probe from the complex in the mixture; and
(b-2) detecting the separated signal probe.

27. The detection method of claim 24, wherein the step (b) of detecting the signal probe comprises steps of:
(b-1) separating only the signal probe from the complex in the mixture; and
(b-2) detecting the separated signal probe,
wherein the step (b-1) of separating only the signal probe comprises the steps of:
(b-1-i) applying the mixture to a magnet to adhere only the complex and a portion of the capture probe not participating in the complex formation to the magnet so as to eliminate other remaining components in the mixture; and
(b-1-ii) separating only the signal probe from the complex adhered to the magnet.

28. The detection method of claim 27, wherein a binding tag is attached to an opposite side of the target-molecule-specific binding region, and
the step (b-2) of detecting the signal probe comprise the steps of:
(b-2-i) applying the separated signal probe to an analytical support, which is coated with a binding partner to the binding tag, to thus immobilize the signal probe via the binding tag onto the analytical support through specific interaction between the binding tag and the binding partner; and
(b-2-ii) detecting a signal of the signal probe immobilized onto the support.

29. The detection method of claim 28, wherein the binding tag is biotin or an analog thereof.

30. The detection method of claim 28, wherein the binding partner is streptavidin or an analog thereof.

31. The detection method of claim 28, wherein an immobilization region, which is a previously known sequence, is further connected adjacent to the signal-generating region, and
the step (b-2-ii) of detecting the signal of the signal probe comprises steps of:
applying an immobilization nucleic acid molecule, to the support, having a single-stranded nucleic acid sequence complementary to the immobilization region and a binding tag that is linked to the sequence and is able to specifically bind to the binding partner; and
detecting the signal of the signal probe.

32. The detection method of claim 31, wherein the binding tag of the immobilization nucleic acid molecule is biotin or an analog thereof.

33. A method of detecting a target protein in a sample intended to be analyzed, comprising the steps of:
(a) treating the sample intended to be analyzed with a signal probe, binding specifically to a target molecule, and a quantified capture competitive molecule, containing a region that binds specifically to a same portion as a portion of a target molecule, to which the signal probe specifically binds, and a magnetic particle that is connected to the region to thus form a complex of the capture competitive molecule and the signal probe, and obtaining a mixture containing the complex as well as a portion of each of the signal probe and the capture probe not participating in the complex formation; and
(b) detecting the signal probe of the complex in the mixture,
wherein the signal probe comprises:
(i) the region that binds specifically to the target protein; and
(ii) a signal-generating region that is linked to the target protein-specific binding region,
wherein the signal-generating region is a double-stranded nucleic acid formed via complementary crosslinking, and the double-stranded nucleic acid contains two or more regions, each of which is labelled with a same or different signal-generating substance,
the step of detecting the signal probe is performed by detecting a signal of the signal-generating region, and
the signal ligand is used in an amount less than an amount capable of detecting both the capture competitive molecule and the target molecule.

34. The detection method of claim 33, wherein the target-molecule-specific binding region is a single-stranded nucleic acid, an antibody, an antigen, or an aptamer.

35. The detection method of claim 33, wherein the step (b) of detecting the signal probe comprises the steps of:
(b-1) separating only the signal probe from the complex in the mixture; and
(b-2) detecting the separated signal probe.

36. The detection method of claim 33, wherein the step (b) of detecting the signal probe comprises the steps of:
(b-1) separating only the signal probe from the complex in the mixture; and
(b-2) detecting the separated signal probe,
wherein the step (b-1) of separating only the signal probe comprises the steps of:
(b-1-i) applying the mixture to a magnet to adhere only the complex and a portion of the capture competitive molecule not participating in the complex formation to the magnet so as to eliminate other remaining components in the mixture; and
(b-1-ii) separating only the signal probe from the complex adhered to the magnet.

37. The detection method of claim 36, wherein a binding tag is attached to an opposite side of the target-molecule-specific binding region, and
the step (b-2) of detecting the signal probe comprise the steps of:
(b-2-i) applying the separated signal probe to an analytical support, which is coated with a binding partner to the binding tag, to thus immobilize the signal probe via the binding tag onto the analytical support through specific interaction between the binding tag and the binding partner; and
(b-2-ii) detecting a signal of the signal probe immobilized onto the support.

38. The detection method of claim 37, wherein the binding tag is biotin or an analog thereof.

39. The detection method of claim 37, wherein the binding partner is streptavidin or an analog thereof.

40. The detection method of claim 37, wherein an immobilization region, which is a previously known sequence, is further connected adjacent to the signal-generating region, and
the step (b-2-ii) of detecting the signal of the signal probe comprises the steps of:
applying, to the support, an immobilization nucleic acid molecule having a single-stranded nucleic acid sequence complementary to the immobilization region and a binding tag that is linked to the sequence and is able to specifically bind to the binding partner; and
detecting the signal of the signal probe.

41. The detection method of claim 40, wherein the binding tag of the immobilization nucleic acid molecule is biotin or an analog thereof.
